# EUROPEAN PATENT APPLICATION

(11) **EP 3 647 423 A1**
(43) Date of publication of application: **06.05.2020**
(21) Application number: 18823662.4
(22) Date of filing: 29.06.2018
(51) Int. Cl.: C12N 15/113, A61K 31/7088, A61K 48/00, A61P 43/00

(54) **HETERO DOUBLE-STRANDED antimiR**

(30) Priority: 30.06.2017 JP 2017129594
(71) Applicant: National University Corporation Tokyo Medical and Dental University, Bunkyo-ku Tokyo 113-8510 (JP)
(72) Inventor: YOKOTA Takanori, Tokyo 113-8510 (JP); YOSHIOKA Kotaro, Tokyo 113-8510 (JP)
(74) Representative: J A Kemp LLP
(86) International application number: PCT/JP2018/024785
(87) International publication number: WO 2019/004420

(57) **Abstract**

Provided is a nucleic acid inhibiting a function of a target miRNA. Provided is a double-stranded nucleic acid complex comprising a first nucleic acid strand of 6 to 30 nucleotide length that hybridizes to a target miRNA to inhibit a function of the target miRNA, and a second nucleic acid strand complementary to the first nucleic acid strand, wherein the first nucleic acid strand is a mixmer comprising a natural nucleoside and a non-natural nucleoside, and the second nucleic acid strand comprises at least one of one or more modified internucleoside linkages and one or more sugar modified nucleosides.

## Description

### Technical Field

The present invention relates to a double-stranded nucleic acid complex inhibiting a function of target microRNA.

### Background Art

MicroRNA (miRNA) is endogenous single-stranded noncoding RNA of approximately 20 to 25 nucleotide length. It is considered that 1000 or more miRNAs are encoded in the human genome. miRNA is involved in the post-transcriptional regulation of gene expression. Typically, miRNA hybridizes to targeted messenger RNA (mRNA) and suppresses protein production via inhibiting translation or the like. miRNA may be involved in various biological processes such as development, cell differentiation, cell proliferation, apoptosis and metabolism.

In recent years, oligonucleotides have attracted attention in the development of medicaments called as nucleic acid drugs. Particularly, the development of nucleic acid drugs exploiting an antisense method has been actively advanced in view of high selectivity of target genes and low toxicity. In general, the antisense method includes a method of selectively altering or inhibiting the expression of a protein encoded by a target gene, comprising introducing, to cells, an oligonucleotide (e.g., an antisense oligonucleotide) complementary to a partial sequence of a mRNA sense strand of the target gene. This method can also inhibit a function of miRNA by introducing an antisense oligonucleotide targeting the miRNA to cells, and thereby allowing the antisense oligonucleotide to bind to the miRNA.

miR-122 is miRNA highly expressed in the liver. A single-stranded antisense oligonucleotide, designated as Miravirsen, targeting miR-122 has been reported (Non Patent Literatures 1 and 2). miR-122 is important for the stability and proliferation of hepatitis C virus (HCV) RNA. It has also been reported that HCV RNA concentrations are lowered by administering the single-stranded antisense oligonucleotide targeting miR-122 to chronic patients infected with HCV genotype 1 (Non Patent Literature 1).

The antisense oligonucleotide targeting miR-21 has been reported to suppress the growth of hepatocellular cancer (Non Patent Literature 3).

The present inventors have developed, as a nucleic acid exploiting the antisense method, a double-stranded nucleic acid complex in which a first nucleic acid strand (antisense oligonucleotide) comprising at least four consecutive nucleotides that are recognized by RNaseH upon hybridization to a transcript, and a complementary strand annealed thereto (Patent Literature 1).

The present inventors have also developed a double-stranded antisense nucleic acid having an exon skipping effect (Patent Literature 2).

The present inventors have also developed a short gapmer antisense oligonucleotide in which additional nucleotides are added to the 5'-terminal or the 3'-terminal, or both of the 5'-terminal and the 3'-terminal of a gapmer (antisense oligonucleotide) (Patent Literature 3), as well as a double-stranded agent for delivering a therapeutic oligonucleotide (Patent Literature 4).

### Citation List

### Patent Literature

Patent Literature 1: International Publication No. WO 2013/089283
Patent Literature 2: International Publication No. WO 2014/203518
Patent Literature 3: International Publication No. WO 2014/132671
Patent Literature 4: International Publication No. WO 2014/192310

### Non Patent Literature

Non Patent Literature 1: Janssen HL et al., Treatment of HCV Infection by Targeting MicroRNA, N. Engl. J. Med., 2013, 368 (18): 1685-1694
Non Patent Literature 2: Elmen J et al., LNA-mediated microRNA silencing in non-human primates. Nature, 2008, 452 (7189): 896-899
Non Patent Literature 3: Wagenaar TR, et al., Anti-miR-21 Suppresses Hepatocellular Carcinoma Growth via Broad Transcriptional Network Deregulation, Mol. Cancer Res., 2015, 13 (6): 1009-1021

### Summary of Invention

### Technical Problem

An object of the present invention is to provide a nucleic acid efficiently inhibiting a function of a target miRNA.

### Solution to Problem

The present inventors have conducted diligent studies to attain the object and consequently completed the present invention by finding that a double-stranded nucleic acid complex comprising a conventional single-stranded mixmer type antisense oligonucleotide that hybridizes to a target miRNA to inhibit a function thereof annealed with a complementary strand comprising at least one of modified internucleoside linkages and sugar modified nucleosides can efficiently inhibit the function of the target miRNA.

Specifically, the present invention encompasses the following embodiments.
[1] A double-stranded nucleic acid complex comprising:
   a first nucleic acid strand of 6 to 30 nucleotide length that hybridizes to a target miRNA to inhibit a function of the target miRNA; and
   a second nucleic acid strand complementary to the first nucleic acid strand, wherein
   the first nucleic acid strand is a mixmer comprising a natural nucleoside and a non-natural nucleoside, and
   the second nucleic acid strand comprises at least one of one or more modified internucleoside linkages and one or more sugar modified nucleosides.
[2] The double-stranded nucleic acid complex according to [1], wherein
   the second nucleic acid strand
   (a) comprises the modified internucleoside linkages consecutively from the 5'-terminal, and comprises the modified internucleoside linkages consecutively from the 3'-terminal;
   (b) comprises the modified internucleoside linkages consecutively from the 5'-terminal, and comprises the sugar modified nucleosides consecutively from the 3'-terminal;
   (c) comprises the sugar modified nucleosides consecutively from the 5'-terminal, and comprises the modified internucleoside linkages consecutively from the 3'-terminal;
   (d) comprises the sugar modified nucleosides consecutively from the 5'-terminal, and comprises the sugar modified nucleosides consecutively from the 3'-terminal; or
   (e) comprises the modified internucleoside linkages and the sugar modified nucleosides consecutively from the 5'-terminal, and comprises the modified internucleoside linkages and the sugar modified nucleosides consecutively from the 3'-terminal.
[3] The double-stranded nucleic acid complex according to [1] or [2], wherein
   the second nucleic acid strand
   comprises at least four modified internucleoside linkages and/or at least four sugar modified nucleosides consecutively from the 5'-terminal,
   comprises at least four modified internucleoside linkages and/or at least four sugar modified nucleosides consecutively from the 3'-terminal, and
   comprises one natural ribonucleoside, or 2 to 8 consecutive natural ribonucleosides linked to each other via phosphodiester linkage(s).
[4] The double-stranded nucleic acid complex according to any one of [1] to [3], wherein at least 50% of internucleoside linkages in the second nucleic acid strand are modified internucleoside linkages.
[5] The double-stranded nucleic acid complex according to any one of [1] to [4], wherein at least 50% of internucleoside linkages in the first nucleic acid strand are modified internucleoside linkages.
[6] The double-stranded nucleic acid complex according to any one of [1] to [5], wherein the modified internucleoside linkages are phosphorothioate linkages.
[7] The double-stranded nucleic acid complex according to any one of [1] to [6], wherein the sugar modified nucleosides comprise 2'-O-methylated sugar.
[8] The double-stranded nucleic acid complex according to any one of [1] to [7], wherein the mixmer is a BNA/DNA mixmer.
[9] The double-stranded nucleic acid complex according to any one of [1] to [8], wherein the second nucleic acid strand further comprises a functional moiety having a function selected from a labeling function, a purification function, and a targeted delivery function.
[10] A pharmaceutical composition comprising a double-stranded nucleic acid complex according to any one of [1] to [9] and a pharmaceutically acceptable carrier.

The present application encompasses the disclosure of Japanese Patent Application No. 2017-129594, to which the present application claims priority.

The present invention provides a nucleic acid efficiently inhibiting a function of a target miRNA.

### Brief Description of Drawings

[Figure 1] Figure 1 is a schematic diagram showing examples of the double-stranded nucleic acid complex according to one embodiment of the present invention.
[Figure 2] Figure 2 is a diagram showing the structures of various natural nucleotides and non-natural nucleotides.
[Figure 3] Figure 3 is a schematic diagram showing the structures of nucleic acid agents used in Example 1. Figure 3 shows. from the left, the name of the nucleic acid agent, the name of an oligonucleotide constituting the nucleic acid agent, and the structure of each nucleic acid agent.
[Figure 4] Figure 4 is a graph showing results of an experiment described in Example 1 and shows the target miRNA (miR-122) suppressive effect of the nucleic acid complex according to a particular embodiment. In the drawing, PBS is for a control. The symbol "****" depicts p < 0.0001. The error bars depict standard deviation.
[Figure 5] Figure 5 is a graph showing the relationship between doses of the nucleic acid agents and relative miR-122 levels, described in Example 2. The error bars depict standard deviation.
[Figure 6] Figure 6 is a photograph showing results of an experiment to evaluate the ability of the nucleic acid agents to bind to a target miRNA, described in Example 3.
[Figure 7] Figure 7 is a graph showing results of an experiment to evaluate the disinhibitory effect of the double-stranded nucleic acid complex targeting miR-122 on a downstream target gene of miR-122, described in Example 4. The symbol "**" depicts p < 0.01. The error bars depict standard deviation.
[Figure 8] Figure 8 is a graph showing results of an experiment to evaluate the influence of the double-stranded nucleic acid complex targeting miR-122 on percent decrease in total serum cholesterol, described in Example 4. The symbol "*" depicts p < 0.05, and the symbol "**" depicts p < 0.01. The error bars depict standard deviation.
[Figure 9] Figure 9 is a graph showing results of an experiment to evaluate the hepatotoxicity of the double-stranded nucleic acid complex according to one embodiment, described in Example 5. The error bars depict standard deviation.
[Figure 10] Figure 10 is a graph showing results of an experiment to evaluate the nephrotoxicity of the double-stranded nucleic acid complex according to one embodiment, described in Example 5. The symbol "*" depicts p < 0.05, and the symbol "**" depicts p < 0.01. The error bars depict standard deviation.
[Figure 11] Figure 11 is a schematic diagram showing the structures of nucleic acid agents used in Example 6. Figure 11 shows, from the left, the name of the nucleic acid agent, the name of an oligonucleotide constituting the nucleic acid agent, and the structure of each nucleic acid agent.
[Figure 12] Figure 12 is a graph showing results of an experiment to evaluate the disinhibitory effect of the double-stranded nucleic acid complex targeting miR-21 on a downstream target gene of miR-21, described in Example 6. The symbol "*" depicts p < 0.05, and the symbol "**" depicts p < 0.01. The error bars depict standard deviation.
[Figure 13] Figure 13 is a schematic diagram showing the structures of nucleic acid agents used in Example 7. Figure 13 shows, from the left, the name of the nucleic acid agent, the name of an oligonucleotide constituting the nucleic acid agent, and the structure of each nucleic acid agent.
[Figure 14] Figure 14 is a graph showing results of an experiment described in Example 7 and shows the target miRNA (miR-122) suppressive effect of the nucleic acid complex according to a particular embodiment. The symbol "**" depicts p < 0.01. The error bars depict standard deviation.
[Figure 15] Figure 15 is a schematic diagram showing the structures of nucleic acid agents used in Example 8. Figure 13 shows, from the left, the name of the nucleic acid agent, the name of an oligonucleotide constituting the nucleic acid agent, and the structure of each nucleic acid agent.
[Figure 16] Figure 16 is a graph showing results of an experiment described in Example 8 and shows the target miRNA (miR-122) suppressive effect of the nucleic acid complex according to a particular embodiment. The symbol "**" depicts p < 0.01. The error bars depict standard deviation. The symbol "n.s." means that there was no significant difference.
[Figure 17] Figure 17 is a schematic diagram showing the structures of nucleic acid agents used in Example 9. Figure 17 shows, from the left, the name of the nucleic acid agent, the name of an oligonucleotide constituting the nucleic acid agent, and the structure of each nucleic acid agent.
[Figure 18] Figure 18 is a graph showing results of an experiment described in Example 9 and shows the target miRNA (miR-122) suppressive effect of the nucleic acid complex according to a particular embodiment. The symbol "**" depicts p < 0.01. The error bars depict standard deviation.
[Figure 19] Figure 19 is a schematic diagram showing the structures of nucleic acid agents used in Example 10. Figure 19 shows, from the left, the name of the nucleic acid agent, the name of an oligonucleotide constituting the nucleic acid agent, and the structure of each nucleic acid agent.
[Figure 20] Figure 20 is a graph showing results of an experiment described in Example 10 and shows the target miRNA (miR-122) suppressive effect of the nucleic acid complex according to a particular embodiment. The symbol "*" depicts p < 0.05, the symbol "**" depicts p < 0.01, and the symbol "****" depicts p < 0.0001. The error bars depict standard deviation.
[Figure 21-1] Figure 21-1 is a schematic diagram showing the structures of nucleic acid agents used in Example 11. Figure 21-1 shows, from the left, the name of the nucleic acid agent, the name of an oligonucleotide constituting the nucleic acid agent, and the structure of each nucleic acid agent.
[Figure 21-2] This drawing is continued sheet of Figure 21-1.
[Figure 22] Figure 22 is a graph showing results of an experiment described in Example 11 and shows the target miRNA (miR-122) suppressive effect of the nucleic acid complex according to a particular embodiment. The symbol "*" depicts p < 0.05, and the symbol "**" depicts p < 0.01. The error bars depict standard deviation.
[Figure 23-1] Figure 23-1 is a schematic diagram showing the structures of nucleic acid agents used in Example 12. Figure 23-1 shows, from the left, the name of the nucleic acid agent, the name of an oligonucleotide constituting the nucleic acid agent, and the structure of each nucleic acid agent.
[Figure 23-2] This drawing is a continued sheet of Figure 23-1.
[Figure 24] Figure 24 is a graph showing results of an experiment described in Example 12 and shows the target miRNA (miR-122) suppressive effect of the nucleic acid complex according to a particular embodiment. The symbol "*" depicts p < 0.05, and the symbol "**" depicts p < 0.01. The error bars depict standard deviation.
[Figure 25] Figure 25 is a schematic diagram showing the structures of nucleic acid agents used in Example 13. Figure 25 shows, from the left, the name of the nucleic acid agent, the name of an oligonucleotide constituting the nucleic acid agent, and the structure of each nucleic acid agent.
[Figure 26] Figure 26 is a graph showing results of an experiment described in Example 13 and shows the target miRNA (miR-122) suppressive effect of the nucleic acid complex according to a particular embodiment. The symbol "*" depicts p < 0.05, and the symbol "**" depicts p < 0.01. The error bars depict standard deviation.
[Figure 27] Figure 27 is a graph showing results of an experiment described in Example 14 and shows the target miRNA (Taf7 mRNA) suppressive effect of the nucleic acid complex according to a particular embodiment. The symbol "*" depicts p < 0.05, and the symbol "**" depicts p < 0.01. The error bars depict standard deviation.

### Description of Embodiments

Hereinafter, the present invention will be described in detail.

### <Nucleic acid complex>

The present invention relates to a nucleic acid complex. The nucleic acid complex comprises a first nucleic acid strand and a second nucleic acid strand complementary to the first nucleic acid strand. The first nucleic acid strand is capable of forming a double-stranded structure by annealing with the second nucleic acid strand to form a double-stranded nucleic acid complex.

As used herein, the term "nucleic acid" is used in the same meaning as a polynucleotide and an oligonucleotide and refers to a polymer of nucleotides having any length.

The term "nucleic acid strand", "nucleotide strand" or "strand" is also used herein to refer to an oligonucleotide.

As used herein, the term "nucleobase" or "base" means a heterocyclic moiety capable of pairing with a base of another nucleic acid. As used herein, the term "complementary" means such a relation that a so-called Watson-Crick base pair (natural base pair) or non-Watson-Crick base pair (Hoogsteen base pair, etc.) can be formed via a hydrogen bond.

In the nucleic acid complex according to one embodiment, the first nucleic acid strand is a nucleotide strand capable of hybridizing to a target microRNA (target miRNA) to inhibit a function of the target miRNA. miRNA is considered to complementarily bind to target messenger RNA (target mRNA) to inhibit the translation of the mRNA into a protein or to induce the degradation of the mRNA, thereby suppressing the expression of the gene. Although not wishing to be bound by any theory, the first nucleic acid strand is capable of inhibiting a function of miRNA by hybridizing to the target miRNA to inhibit the binding of the miRNA to mRNA. The first nucleic acid strand may inhibit a function of miRNA by hybridizing to the target miRNA to reduce the amount (level) of the target miRNA.

As used herein, the first nucleic acid strand is also referred to as an "antisense oligonucleotide", a "miRNA antisense oligonucleotide" or an "antisense nucleic acid".

As used herein, the term "antisense effect" refers to the suppression of the level of a target miRNA or the inhibition of a function of a target miRNA resulting from the hybridization between the target miRNA and a strand complementary to at least a partial sequence of the miRNA.

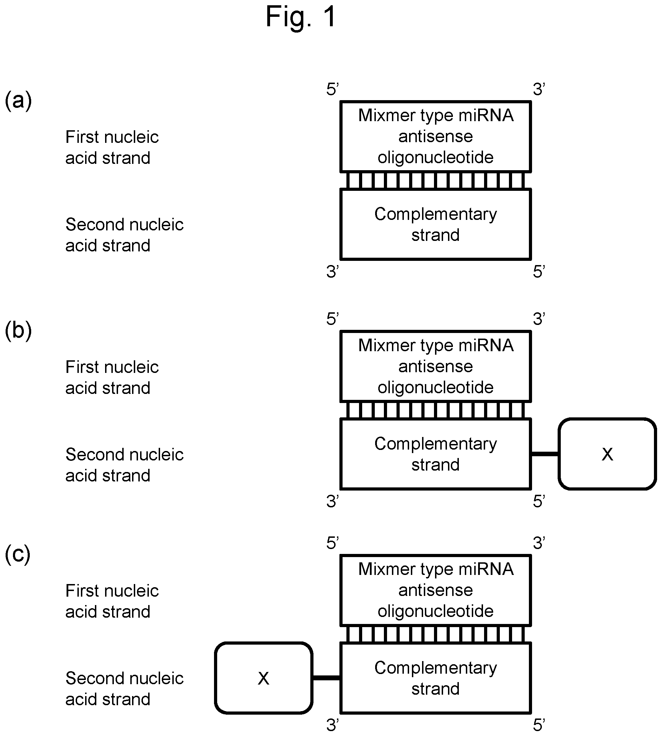

A schematic diagram of the nucleic acid complex according to one embodiment of the present invention is shown in Figures 1a to 1c. The nucleic acid complex can comprise the first nucleic acid strand and the second nucleic acid strand (Figure 1a). As mentioned later, at least one functional moiety "X" may be linked to either of the 5'-terminal or the 3'-terminal, or both, of the second nucleic acid strand in the nucleic acid complex. Figure 1b shows a schematic diagram of the nucleic acid complex with the functional moiety "X" linked to the 5'-terminal of the second nucleic acid strand. Figure 1c shows a schematic diagram of the nucleic acid complex with the functional moiety "X" linked to the 3'-terminal of the second nucleic acid strand.

MicroRNA (miRNA) is single-stranded noncoding RNA of approximately 20 to 25 nucleotide length. The miRNA pathway can be described as follows: a gene of miRNA on the genome is transcribed into single-stranded RNA of several hundreds to several thousands of nucleotide length. The RNA thus obtained by transcription forms a stem-loop structure and becomes pri-miRNA (primary miRNA). Subsequently, the pri-miRNA molecule is partially cleaved by a RNaseIII-like enzyme (Drosha) in the nucleus to generate pre-miRNA (precursor miRNA) of approximately 70 nucleotide length having a stem-loop structure. Subsequently, the pre-miRNA molecule is transported from within the cellular nucleus to the cytoplasm by a transporter protein (Exportin-5). Subsequently, the pre-miRNA is cleaved by another RNaseIII enzyme (Dicer) in the cytoplasm to generate double-stranded miRNA. The double-stranded miRNA is incorporated into an RNA-induced silencing complex (RISC) comprising Argonaute protein. The double-stranded miRNA thus incorporated into RISC becomes two single-stranded molecules in the RISC where a more unstable single strand is degraded while the remaining single-stranded miRNA becomes mature miRNA. It is considered that the mature miRNA binds to mRNA having a complementary nucleotide sequence and thereby inhibits the translation of the mRNA, or induces the degradation of the mRNA and thereby suppresses the expression of each gene.

The target miRNA may be any miRNA. The target miRNA can be mature miRNA. Examples of the target miRNA include miR-122, miR-21, miR-98, miR-34c, miR-155, miR-34, Let-7, miR-208, miR-195, miR-221, miR-103, miR-105, and miR-10b (see, for example, Li Z & Rana TM, Nature Reviews Drug Discovery, 2014, 13: 622-638). The nucleotide sequence of microRNA can be obtained from available databases, for example, the NCBI (National Center for Biotechnology Information, USA) database, and the miRBase database (Kozomara A, Griffiths-Jones S. NAR 2014 42: D68-D73; Kozomara A, Griffiths-Jones S. NAR 2011 39: D152-D157; Griffiths-Jones S, Saini HK, van Dongen S, Enright AJ. NAR 2008 36: D154-D158; Griffiths-Jones S, Grocock RJ, van Dongen S, Bateman A, Enright AJ. NAR 2006 34: D140-D144; and Griffiths-Jones S. NAR 2004 32: D109-D111).

The organism from which the target miRNA is derived may also be any organism. The organism can be, for example, a mammal, for example, a primate (e.g., a cynomolgus monkey, a chimpanzee and a human) or a nonprimate (e.g., cattle, a pig, sheep, a horse, a cat, a dog, a guinea pig, a rat and a mouse) and is preferably a human.

The nucleotide sequence of mouse miR-122 is shown in SEQ ID NO: 1. The nucleotide sequence of human miR-122 is the same as that of the mouse. The nucleotide sequence of mouse miR-21 is shown in SEQ ID NO: 2. The nucleotide sequence of human miR-21 is the same as that of the mouse.

The first nucleic acid strand may comprise a nucleotide sequence capable of hybridizing to at least a portion of the target miRNA. The first nucleic acid strand may comprise a nucleotide sequence capable of hybridizing to a nucleotide sequence from positions 1 to 20 (the 1st to 20th nucleotides counted from the 5'-terminal of the miRNA), positions 1 to 18, positions 2 to 17, positions 2 to 16, positions 2 to 14, positions 2 to 12, positions 2 to 10, or positions 2 to 8 of the miRNA. In one embodiment, the first nucleic acid strand can comprise a nucleotide sequence capable of hybridizing to a nucleotide sequence from positions 2 to 16 of the miRNA.

The first nucleic acid strand is not necessarily required to comprise a nucleotide sequence completely complementary to at least a portion of the target miRNA and can comprise a nucleotide sequence complementary thereto by at least 70%, preferably at least 80%, more preferably at least 90% (e.g., 95% or more). The sequence complementarity can be determined by using a BLAST program or the like. The first nucleic acid strand is capable of hybridizing to the target miRNA when their sequences are complementary to each other.

The hybridization conditions may be stringent conditions, for example, low stringent conditions or highly stringent conditions. The low stringent conditions can be, for example, 30°C, 2 × SSC, and 0.1% SDS. The highly stringent conditions can be, for example, 65°C, 0.1 × SSC, and 0.1% SDS. The hybridization stringency can be adjusted by changing conditions such as temperature and salt concentration. In this context, 1 × SSC contains 150 mM sodium chloride and 15 mM sodium citrate.

Those skilled in the art can readily determine the conditions (temperature, salt concentration, etc.) under which two strands are capable of hybridizing to each other, in consideration of the degree of complementarity between the strands. Those skilled in the art can also readily design the antisense nucleic acid (first nucleic acid strand) complementary to at least a portion of the target miRNA, for example, on the basis of information on the nucleotide sequence of the target miRNA.

The second nucleic acid strand is complementary to the first nucleic acid strand, as a rule. However, the second nucleic acid strand is not necessarily required to comprise a nucleotide sequence completely complementary to the first nucleic acid strand and can comprise a nucleotide sequence complementary thereto by at least 70%, preferably at least 80%, more preferably at least 90% (e.g., 95% or more). The sequence complementarity can be determined by using a BLAST program or the like. The first nucleic acid strand and the second nucleic acid strand are capable of annealing with each other when their sequences are complementary to each other. Those skilled in the art can readily determine the conditions (temperature, salt concentration, etc.) under which two nucleic acid strands can anneal with each other.

The lower limits of the base lengths of the first nucleic acid strand and the second nucleic acid strand can be, but not limited to, each independently 6 nucleotide length, 7 nucleotide length, 8 nucleotide length, 9 nucleotide length, 10 nucleotide length, 11 nucleotide length, 12 nucleotide length, 13 nucleotide length, 14 nucleotide length or 15 nucleotide length. The upper limits of the base lengths of the first nucleic acid strand and the second nucleic acid strand can be each independently 30 nucleotide length, 25 nucleotide length, 24 nucleotide length, 23 nucleotide length, 22 nucleotide length, 21 nucleotide length, 20 nucleotide length, 19 nucleotide length, 18 nucleotide length, 17 nucleotide length or 16 nucleotide length. The specific ranges of the base lengths of the first nucleic acid strand and the second nucleic acid strand can be each independently, for example, 6 to 30 nucleotide length, 8 to 25 nucleotide length, 10 to 20 nucleotide length, 12 to 18 nucleotide length, or 14 to 16 nucleotide length. The first nucleic acid strand and the second nucleic acid strand may have the same length or may have different lengths (e.g., lengths differing by 1 to 3 bases). The duplex structure formed by the first nucleic acid strand and the second nucleic acid strand may comprise a bulge. In a particular embodiment, the choice of the length depends on, for example, cost and synthesis yields as well as the intensity of the inhibition of a miRNA function, and the specificity of the nucleic acid strand for the target miRNA.

In general, a "nucleoside" is a combination of a base and a sugar. The nucleic acid base (known as a base) moiety of a nucleoside is usually a heterocyclic base moiety. A "nucleotide" further comprises a phosphate group covalently bound to the sugar moiety of the nucleoside. In a nucleoside comprising a pentofuranosyl sugar, a phosphate group can be linked to the 2', 3', or 5' hydroxyl moiety of the sugar. Typically, the 3' position of a sugar is linked to the 5' position of an adjacent sugar via phosphoester bond. An oligonucleotide is formed by covalent bonds between nucleosides adjacent to each other, forming a linear polymer oligonucleotide. In general, phosphate groups are considered to form internucleoside linkages of an oligonucleotide inside the oligonucleotide structure.

Herein, a nucleic acid strand may be constituted by a natural nucleotide and/or an unnatural nucleotide. Herein, a "natural nucleotide" comprises a deoxyribonucleotide found in DNA and a ribonucleotide found in RNA. Herein, "deoxyribonucleotide" and "ribonucleotide" may be referred to as "DNA nucleotide" and "RNA nucleotide" respectively.

A "natural nucleoside" as used herein comprises a deoxyribonucleoside found in DNA and a ribonucleoside found in RNA. Herein, "deoxyribonucleoside" and "ribonucleoside" may be referred to as "DNA nucleoside" and "RNA nucleoside" respectively.

An "unnatural nucleotide" refers to any nucleotide other than a natural nucleotide and encompasses a modified nucleotide and a nucleotide mimic. Similarly, an "unnatural nucleoside" as used herein refers to any nucleoside other than a natural nucleoside and encompasses a modified nucleoside and a nucleoside mimic. Herein, a "modified nucleotide" refers to a nucleotide having any one or more of a modified sugar moiety, a modified internucleoside linkage, and a modified nucleic acid base. Herein, a "modified nucleoside" refers to a nucleoside having a modified sugar moiety and/or a modified nucleic acid base. A nucleic acid strand comprising an unnatural oligonucleotide often has desirable characteristics that allow, for example, enhanced cell uptake, enhanced affinity to a nucleic acid target, increased stability in the presence of nuclease, or increased inhibitory activity, and accordingly is more preferable than a natural type.

Herein, a "modified internucleoside linkage" refers to an internucleoside linkage having a substitution or any change from a naturally-occurring internucleoside linkage (in other words, phosphodiester linkage). A modified internucleoside linkage encompasses an internucleoside linkage comprising a phosphorus atom and an internucleoside linkage comprising no phosphorus atom. Representative examples of phosphorus-containing internucleoside linkages include, but are not limited to, a phosphodiester linkage, phosphorothioate linkage, phosphorodithioate linkage, phosphotriester linkage, methylphosphonate linkage, methylthiophosphonate linkage, boranophosphate linkage, and phosphoramidate linkage. A phosphorothioate linkage refers to an internucleoside linkage resulting from a phosphodiester linkage whose non-bridged oxygen atom is substituted with a sulfur atom. Methods of preparing phosphorus-containing and non-phosphorus-containing linkages are well known. Modified internucleoside linkages are preferably those having a higher nuclease resistance than naturally occurring internucleoside linkages. Internucleoside linkages having a higher nuclease resistance than naturally occurring internucleoside linkages are known to the skilled person.

Herein, a "modified nucleic acid base" or "modified base" refers to any nucleic acid base other than adenine, cytosine, guanine, thymine, or uracil. An "unmodified nucleic acid base" or "unmodified base" (natural nucleic acid base) refers to adenine (A) and guanine (G) which are purine bases and to thymine (T), cytosine (C), and uracil (U) which are pyrimidine bases. Examples of modified nucleic acid bases include, but are not limited to: 5-methylcytosine, 5-fluorocytosine, 5-bromocytosine, 5-iodocytosine, or N4-methylcytosine; 5-fluorouracil, 5-bromouracil or 5-iodouracil; 2-thiothymine; N6-methyladenine or 8-bromoadenine; and N2-methylguanine or 8-bromoguanine etc.

Herein, a "modified sugar" refers to a sugar having a substitution and/or any change from a natural sugar moiety (in other words, a sugar moiety found in DNA (2'-H) or RNA (2'-OH)). Herein, a nucleic acid strand may optionally comprise a sugar-modified nucleoside. The "sugar-modified nucleoside" refers to a modified nucleoside comprising a modified sugar. The sugar-modified nucleoside can confer enhanced nuclease stability, an increased binding affinity, or any other useful biological characteristics to a nucleic acid strand.

In a specific embodiment, a nucleoside comprises a chemically-modified ribofuranose ring moiety. Examples of chemically-modified ribofuranose rings include, but are not limited to, those resulting from: addition of a substituent (including 5' or 2' substituents); formation of a bicyclic nucleic acid (bridged nucleic acid, or BNA) by bridge-formation of non-geminal ring atoms; substitution of a ribosyl ring oxygen atom with S, N(R), or C(R1)(R2) (R, R1, and R2 independently represent H, C₁-C₁₂ alkyl, or a protecting group, respectively); and combinations thereof.

Examples of suga-modified nucleosides include, but are not limited to, nucleosides comprising a 5'-vinyl, 5'-methyl(R or S), 4'-S, 2'-F (2'-fluoro group), 2'-OCH₃ (2'-OMe group or 2'-O-methyl group), and 2'-O(CH₂)₂OCH₃ (2'-O-MOE group) substituent. The substituent at the 2' position can be selected from allyl, amino, azido, thio, -O-allyl, -O-C₁-C₁₀ alkyl, -OCF₃, -O(CH₂)₂SCH₃, -O(CH₂)₂-O-N(Rm)(Rn), and -O-CH₂-C(=O)-N(Rm)(Rn), and each of Rm and Rn independently represents H or a substituted or unsubstituted C₁-C₁₀ alkyl. Herein, a "2'-modified sugar" refers to a furanosyl sugar modified at the 2' position. The 2'-modified sugar includes, for example, 2'-O-methylated sugar.

Further examples of the suga-modified nucleosides include a bicyclic nucleoside. As used herein, a "bicyclic nucleoside" refers to a modified nucleoside comprising a bicyclic sugar moiety. In general, a nucleic acid comprising a bicyclic sugar moiety is referred to as a bridged nucleic acid (BNA). Herein, a nucleoside comprising a bicyclic sugar moiety may be referred to as a "bridged nucleoside".

A bicyclic sugar may be a sugar in which the 2' position carbon atom and 4' position carbon atom are bridged by two or more atoms. Examples of bicyclic sugars are known to a person skilled in the art. One subgroup of a nucleic acid comprising a bicyclic sugar (BNA) can be described as having a 2' position carbon atom and 4' position carbon atom that are bridged by 4'-(CH₂)ₚ-O-2', 4'-(CH₂)ₚ-CH₂-2', 4'-(CH₂)ₚ-S-2', 4'-(CH₂)ₚ-OCO-2', or 4'-(CH₂)ₙ₋N(R₃)-O-(CH₂)ₘ-2' [wherein p, m, and n represent an integer of 1 to 4, an integer of 0 to 2, and an integer of 1 to 3 respectively; R₃ represents a hydrogen atom, alkyl group, alkenyl group, cycloalkyl group, aryl group, aralkyl group, acyl group, sulfonyl group, and unit substituent (fluorescently or chemiluminescently labeled molecule, functional group having nucleic acid cleaving activity, intracellular or intranuclear localization signal peptide, or the like)]. Furthermore, regarding BNA according to a specific embodiment, in the OR₂ substituent at the 3' position carbon atom and the OR₁ substituent at the 5' position carbon atom, R₁ and R₂ are typically hydrogen atoms and may be the same or different, and in addition, may be a protecting group for a hydroxyl group for nucleic acid synthesis, alkyl group, alkenyl group, cycloalkyl group, aryl group, aralkyl group, acyl group, sulfonyl group, silyl group, phosphate group, phosphate group protected by a protecting group for nucleic acid synthesis, or -P(R₄)R₅ [wherein R₄ and R₅ are the same as or different from each other, and each represent a hydroxyl group, hydroxyl group protected by a protecting group for nucleic acid synthesis, mercapto group, mercapto group protected by a protecting group for nucleic acid synthesis, amino group, C₁-C₅ alkoxy group, C₁-C₅ alkylthio group, C₁-C₆ cyanoalkoxy group, or amino group substituted with a C₁-C₅ alkyl group]. Non-limiting examples of such BNAs include: methyleneoxy (4' -CH₂-O-2') BNA (LNA (Locked Nucleic Acid ®, also known as 2',4'-BNA), for example, α-L-methyleneoxy (4' -CH₂-O-2') BNA or β-D-methyleneoxy (4'-CH₂-O-2') BNA; ethyleneoxy (4'-(CH₂)₂-O-2') BNA (also known as ENA); β-D-thio(4'-CH₂-S-2') BNA; aminooxy(4'-CH₂-O-N(R₃)-2') BNA; oxyamino(4'-CH₂-N(R₃)-O-2') BNA (also known as 2',4'-BNA^{NC}); 2',4'-BNA^{coc}; 3'-amino-2',4'-BNA; 5'-methyl BNA; (4'-CH(CH₃)-O-2') BNA (also known as cEt BNA); (4'-CH(CH₂OCH₃)-O-2') BNA (also known as cMOE BNA); amide BNA (4'-C(O)-N(R)-2') BNA (R = H or Me) (also known as AmNA); and other BNAs known to a person skilled in the art.

Herein, a bicyclic nucleoside having a methyleneoxy(4'-CH₂-O-2') bridge may be referred to as an LNA nucleoside.

Methods of preparing a modified sugar are well known to a person skilled in the art. In a nucleotide having a modified sugar moiety, a nucleic acid base moiety (natural one, modified one, or a combination thereof) may be maintained for hybridization with a suitable nucleic acid target.

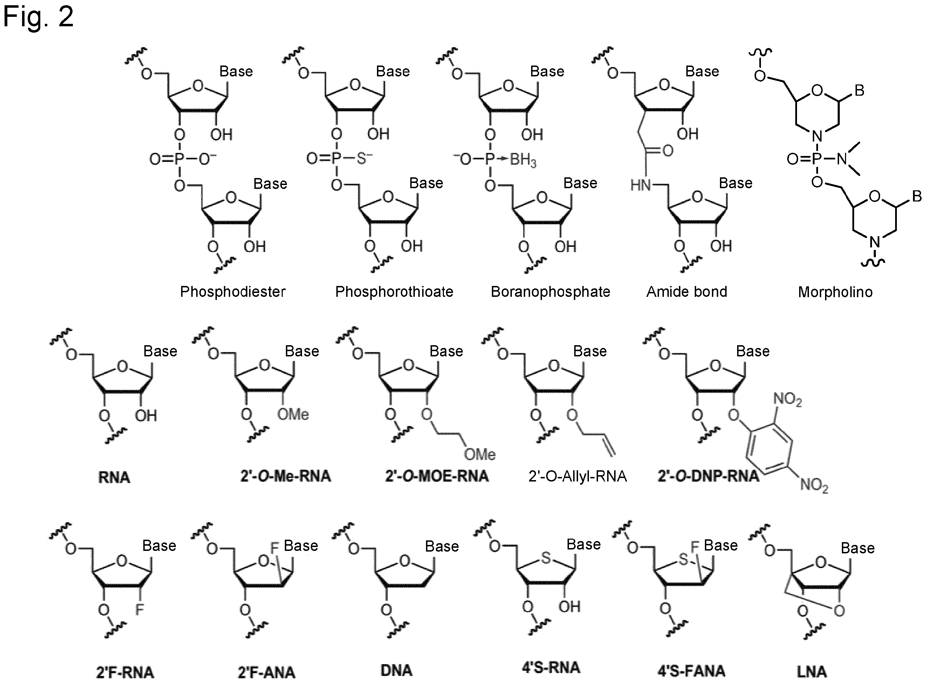

Herein, a "nucleoside mimic" comprises, at one or more positions in an oligomer compound, a sugar, or a sugar and a base, and optionally a structure used to substitute a linkage. An "oligomer compound" refers to a polymer of linked monomer subunits capable of hybridizing with at least a region of a nucleic acid molecule. Examples of nucleoside mimics include morpholino, cyclohexenyl, cyclohexyl, tetrahydropyranyl, bicyclic, or tricyclic sugar mimics, for example, nucleoside mimics having a non-furanose sugar unit. A "nucleotide mimic" comprises, at one or more positions in an oligomer compound, a nucleoside and a structure used to substitute a linkage. Examples of nucleotide mimics include peptide nucleic acids or morpholino nucleic acids (morpholinos linked by -N(H)-C(=O)-O- or another non-phosphodiester linkage). A peptide nucleic acid (PNA) is a nucleotide mimic having a main-chain to which N-(2-aminoethyl)glycine instead of a sugar is linked by an amide bond. An example of the structure of a morpholino nucleic acid is shown in Figure 2. A "mimic" refers to a group that substitutes at least one of a sugar, nucleic acid base, and internucleoside linkage. In general, a mimic is used instead of a sugar or a combination of a sugar and an internucleoside linkage, and a nucleic acid base is maintained for hybridization with a selected target.

In general, modification can be carried out so that nucleotides in the same strand can independently be modified differently. To provide resistance to enzymic cleavage, the same nucleotide can have a modified internucleoside linkage (for example, a phosphorothioate linkage) and further have a modified sugar (for example, a 2'-O-methyl modified sugar or a bicyclic sugar). The same nucleotide can also have a modified nucleic acid base (for example, 5-methylcytosine) and further have a modified sugar (for example, a 2'-O-methyl modified sugar or a bicyclic sugar). The same nucleotide can also have a modified internucleoside linkage (for example, a phosphorothioate linkage), have a modified sugar (for example, a 2'-O-methyl modified sugar or a bicyclic sugar), and further have a modified nucleic acid base (for example, 5-methylcytosine).

The number, kind, and position of unnatural nucleotides in a nucleic acid strand can have an impact on an antisense effect and the like provided by the nucleic acid complex according to the present invention. The selection of a modification can vary, for example, depending on the sequence of a target mRNA, but a person skilled in the art can determine a suitable embodiment by reference to the explanation in documents related to an antisense method (for example, WO2007/143315, WO2008/043753, and WO 2008/049085). Furthermore, a related modification can be evaluated in a case where an antisense effect of a nucleic acid complex obtained after modification is measured, and where a measured value thus obtained is not significantly lower than a measured value of a nucleic acid complex existing before modification (for example, in a case where a measured value obtained after modification is 70% or more, 80% or more, or 90% or more of a measured value of a nucleic acid complex existing before modification).

Measurement of an antisense effect can be carried out by introducing a test nucleic acid compound into a cell or a subject (for example, a mouse), or the like, and then suitably using a known technique such as Northern blotting and quantitative PCR to thereby measure the expression level of a target miRNA, for example, as described in Examples below.

The measurement of an antisense effect may be performed by introducing a test nucleic acid compound into a subject (for example, mouse), and measuring the expression level of a target miRNA in a target organ (for example, liver) of the subject.

It is shown that the test nucleic acid compound can produce an antisense effect, in cases where the measured expression level of a target miRNA is reduced by at least 20%, at least 30%, at least 40%, or at least 50% as compared to a negative control (for example, a vehicle-administration or a no-treatment). The nucleic acid complex according to one embodiment can have a higher (for example, two or more times higher) antisense effect than that provided by the first nucleic acid strand alone.

Alternatively, the measurement of the antisense effect may be carried out by introducing a test nucleic acid compound into a cell or a subject (for example, a mouse), or the like, and then suitably using a known technique such as Northern blotting, quantitative PCR, and western blotting to thereby measure the level of a target gene regulated by miRNA, or the level of a protein translated from the gene.

It is shown that the test nucleic acid compound can produce an antisense effect, in cases where the measured expression level of a miRNA or protein is increased by at least 20%, at least 30%, at least 40%, or at least 50% as compared to a negative control (for example, a vehicle-administration or a no-treatment).

The nucleosides constituting the first nucleic acid strand can be natural nucleosides (natural deoxyribonucleosides or natural ribonucleosides, or both) and/or non-natural nucleosides.

In one embodiment, the nucleoside constitution of the first nucleic acid strand is a mixmer. As used herein, the "mixmer" means a nucleic acid strand constituted by periodic nucleosides or random segment lengths of alternating nucleosides. The mixmer can comprise a natural nucleoside (e.g., a natural deoxyribonucleoside) and a non-natural nucleoside (e.g., a bicyclic nucleoside, preferably an LNA nucleoside). The mixmer comprising a natural deoxyribonucleoside and a bridged nucleoside is referred to herein as a "BNA/DNA mixmer". The bridged nucleoside may comprise a modified nucleobase (e.g., 5-methylcytosine). The mixmer comprising a natural deoxyribonucleoside and an LNA nucleoside is referred to herein as an "LNA/DNA mixmer". The mixmer is not necessarily required to be limited by comprising only two nucleoside species. The mixmer can comprise any number of nucleoside species, irrespective of whether or not the nucleoside is a natural or modified nucleoside or nucleoside mimic.

In one embodiment, the mixmer does not comprise four or more consecutive natural nucleosides. The mixmer may not comprise three or more consecutive natural nucleosides. In another embodiment, the mixmer does not comprise three or more or four or more consecutive natural deoxyribonucleosides. In a further alternative embodiment, the mixmer does not comprise three or more or four or more consecutive natural ribonucleosides.

In one embodiment, the mixmer does not comprise three or more or four or more consecutive non-natural nucleosides. In another embodiment, the mixmer does not comprise three or more or four or more consecutive bicyclic nucleosides (e.g., LNA nucleosides).

Internucleoside linkages in the first nucleic acid strand can be naturally occurring internucleoside linkages and/or modified internucleoside linkages.

The first nucleic acid strand may comprise at least 1, at least 2, at least 3, at least 4, at least 5, at least 6, at least 7, at least 8, at least 9, or at least 10 modified internucleoside linkages. The first nucleic acid strand may comprise at least 1, at least 2, at least 3, at least 4, or at least 5 modified internucleoside linkages consecutively from the 5'-terminal. The first nucleic acid strand may comprise at least 1, at least 2, at least 3, at least 4, or at least 5 modified internucleoside linkages consecutively from the 3'-terminal. As used herein, for example, the phrase "comprise two modified internucleoside linkages consecutively from the 5'-terminal" means that an internucleoside linkage most proximal to the 5'-terminal, and an internucleoside linkage positioned adjacent thereto in a direction toward the 3'-terminal are modified internucleoside linkages. Such terminal modified internucleoside linkages are preferred because they can inhibit undesired degradation of the nucleic acid strand.

In one embodiment, at least 50%, at least 60%, at least 70%, at least 80%, at least 90%, or 100% of internucleoside linkages in the first nucleic acid strand may be modified internucleoside linkages. The modified internucleoside linkages may be phosphorothioate linkages.

Nucleosides constituting the second nucleic acid strand can be natural nucleosides (deoxyribonucleosides or ribonucleosides, or both) and/or non-natural nucleosides.

The second nucleic acid strand can comprise at least one of one or more modified internucleoside linkages and one or more sugar modified nucleosides. Specifically, the second nucleic acid strand may comprise one or more modified internucleoside linkages, may comprise one or more sugar modified nucleosides, or may comprise both of one or more modified internucleoside linkages and one or more sugar modified nucleosides. The modified internucleoside linkages may be phosphorothioate linkages. The sugar modified nucleosides may comprise 2'-modified sugar, for example, 2'-O-methylated sugar.

The second nucleic acid strand may comprise at least 1, at least 2, at least 3, at least 4, at least 5, at least 6, at least 7, at least 8, at least 9, or at least 10 modified internucleoside linkages. The second nucleic acid strand may comprise at least 1, at least 2, at least 3, at least 4, or at least 5 modified internucleoside linkages consecutively from the 5'-terminal. The second nucleic acid strand may comprise at least 1, at least 2, at least 3, at least 4, or at least 5 modified internucleoside linkages consecutively from the 3'-terminal.

At least 50%, at least 60%, at least 70%, at least 80%, at least 90%, or 100% of internucleoside linkages in the second nucleic acid strand may be modified internucleoside linkages.

The second nucleic acid strand may comprise at least 1, at least 2, at least 3, at least 4, at least 5, at least 6, at least 7, at least 8, at least 9, or at least 10 sugar modified nucleosides. The second nucleic acid strand may comprise at least 1, at least 2, at least 3, at least 4, or at least 5, for example, 1 to 5 or 1 to 3 sugar modified nucleosides consecutively from the 5'-terminal. The second nucleic acid strand may comprise at least 1, at least 2, at least 3, at least 4, or at least 5, for example, 1 to 5 or 1 to 3 sugar modified nucleosides consecutively from the 3'-terminal. As used herein, for example, the phrase "comprise two sugar modified nucleosides consecutively from the 5'-terminal" means that a nucleoside positioned closest to the 5'-terminal, and a nucleoside positioned adjacent thereto in a direction toward the 3'-terminal are sugar modified nucleosides. Such terminal sugar modified nucleosides are preferred because they can inhibit undesired degradation of the nucleic acid strand.

In one embodiment, the second nucleic acid strand may
comprise at least one modified internucleoside linkage consecutively from the 5'-terminal, and comprise at least one modified internucleoside linkage consecutively from the 3'-terminal,
comprise at least one modified internucleoside linkage consecutively from the 5'-terminal, and comprise at least one sugar modified nucleoside consecutively from the 3'-terminal,
comprise at least one sugar modified nucleoside consecutively from the 5'-terminal, and comprise at least one modified internucleoside linkage consecutively from the 3'-terminal, or
comprise at least one sugar modified nucleoside consecutively from the 5'-terminal, and comprise at least one sugar modified nucleoside consecutively from the 3'-terminal.

In a further embodiment, the second nucleic acid strand may
comprise at least one modified internucleoside linkage consecutively from the 5'-terminal, and comprise at least one modified internucleoside linkage and at least one sugar modified nucleoside consecutively from the 3'-terminal,
comprise at least one sugar modified nucleoside consecutively from the 5'-terminal, and comprise at least one modified internucleoside linkage and at least one sugar modified nucleoside consecutively from the 3'-terminal,
comprise at least one modified internucleoside linkage and at least one sugar modified nucleoside consecutively from the 5'-terminal, and comprise at least one modified internucleoside linkage consecutively from the 3'-terminal, or
comprise at least one modified internucleoside linkage and at least one sugar modified nucleoside consecutively from the 5'-terminal, and comprise at least one sugar modified nucleoside consecutively from the 3'-terminal.

In a further embodiment, the second nucleic acid strand may
comprise at least one modified internucleoside linkage and at least one sugar modified nucleoside consecutively from the 5'-terminal, and
comprise at least one modified internucleoside linkage and at least one sugar modified nucleoside consecutively from the 3'-terminal.

The second nucleic acid strand may comprise at least 1, at least 2, at least 3, at least 4, or at least 5 (e.g., consecutive) natural ribonucleosides. The second nucleic acid strand may comprise 1 to 15, 1 to 13, 1 to 9, 1 to 8, 2 to 7, 3 to 6 or 4 or 5 (e.g., consecutive) natural ribonucleosides. The consecutive natural ribonucleosides may be linked to each other via phosphodiester linkage(s), or may be linked to each other via modified internucleoside linkage(s). At least 30%, at least 40%, at least 50%, at least 60%, at least 70%, at least 80%, at least 90%, or 100% of nucleosides constituting the second nucleic acid strand may be natural nucleosides (e.g., natural ribonucleosides).

In a particular embodiment, the second nucleic acid strand may
comprise at least four modified internucleoside linkages and/or at least four sugar modified nucleosides consecutively from the 5'-terminal,
comprise at least four modified internucleoside linkages and/or at least four sugar modified nucleosides consecutively from the 3'-terminal, and
comprise one natural ribonucleoside, or 2 to 8 consecutive natural ribonucleosides linked to each other via phosphodiester linkage(s) (e.g., at a site other than those described above, for example, at a nonterminal site). The second nucleic acid strand may comprise, for example, 3 to 7, 4 to 6, or 5 consecutive natural ribonucleosides (e.g., at a site other than those described above, for example, at a nonterminal site) linked to each other via phosphodiester linkages.

The second nucleic acid strand may comprise any combination of the modified internucleoside linkages and the sugar modified nucleosides.

The second nucleic acid strand can be cleavable by an enzyme *in vivo.* Also, the second nucleic acid strand can efficiently hybridize to the first nucleic acid strand before cleavage and efficiently dissociate from the first nucleic acid strand after cleavage. The second nucleic acid strand can comprise a non-natural nucleoside having binding affinity appropriate for achieving such hybridization and dissociation.

The second nucleic acid strand may have a structure advantageous for dissociation from the first nucleic acid strand after cleavage *in vivo.* The structure can comprise, for example, a modified nucleotide advantageous for dissociation. The structure can comprise, for example, a modified internucleoside linkage advantageous for dissociation. The structure can comprise, for example, a sequence that is not complementary to the first nucleic acid strand. Non-limiting examples of the structure include mismatch and bulge structures.

In an embodiment, the second nucleic acid strand can comprise at least one "functional moiety (X)" linked to a polynucleotide. The functional moiety may be linked to the 5' end (Figure 1b) or the 3' end (Figure 1c) of the second nucleic acid strand. Alternatively, the functional moiety may be linked to a nucleotide in the interior part of the polynucleotide. In other embodiments, the second nucleic acid strand comprises two or more functional moieties, which may be linked to the polynucleotide at multiple positions and/or to the polynucleotide at one position as a group.

The linkage between the second nucleic acid strand and the functional moiety may be a direct linkage or an indirect linkage mediated by another material. However, in a particular embodiment, preferably a functional moiety is directly linked to the second nucleic acid strand via, for example, covalent bonding, ionic bonding, and/or hydrogen bonding, more preferably via covalent bonding considering that more stable linkages can be obtained. A functional moiety may also be linked to the second nucleic acid strand via a cleavable linking group. For example, a functional moiety may be linked via a disulfide bond.

The structure of the "functional moiety" according to a particular embodiment is not limited to a particular one as long as the functional moiety confers a desired function to a nucleic acid complex and/or a strand to which the functional moiety is linked. Examples of desired functions include a labeling function, a purification function, and a delivery function. Examples of a moiety giving a labeling function include a compound such as a fluorescent protein and a luciferase. Examples of moieties which give a purification function include a compound such as biotin, avidin, His-tag peptide, GST-tag peptide, and FLAG-tag peptide etc.

In some embodiments, a functional moiety serves to enhance transport to cells. For example, particular peptide tags are demonstrated to enhance cellular uptake of oligonucleotides, when conjugated to the oligonucleotides. Examples of such peptide tags include the arginine-rich peptide P007 and B peptides disclosed in HaiFang Yin et al., Human Molecular Genetics, Vol. 17 (24), 3909-3918 (2008) and references cited therein.

Furthermore, the second nucleic acid strand is preferably linked with, as a functional moiety, a molecule having an activity to deliver a nucleic acid complex according to some embodiments of the present invention to a "target site" in the body, in order to deliver a nucleic acid complex (or the first nucleic acid strand) according to the present invention to a target site or a target region in the body with high specificity and high efficiency and thereby very effectively inhibit the expression of a target miRNA from a related nucleic acid.

A moiety having a "targeted delivery function" may be, for example, a lipid, to be capable of delivering a nucleic acid complex according to a particular embodiment of the present invention to, for example, the liver with high specificity and high efficiency. Examples of such lipids include lipids such as cholesterol and fatty acids (for example, vitamin E (tocopherol, tocotrienol), vitamin A, and vitamin D); lipophilic vitamins such as vitamin K (for example, acylcarnitine); intermediate metabolites such as acyl-CoA; glycolipids, glycerides, and derivatives thereof. However, among these, cholesterol and vitamin E (tocopherol and tocotrienol) are used in a particular embodiment, considering that these compounds have higher safety. However, a nucleic acid complex according to a particular embodiment of the present invention may not be linked with a lipid.

Furthermore, examples of the "functional moiety" according to a particular embodiment include sugars (for example, glucose and sucrose), since it can deliver a nucleic acid complex according to a particular embodiment of the present invention to the brain with high specificity and high efficiency.

Additionally, examples of the "functional moiety" according to a particular embodiment include peptides or proteins (for example, receptor ligands, and antibodies and/or fragments thereof), since it can bind to various proteins present on the surface of cells in various organs and thereby deliver the nucleic acid complex according to a particular embodiment of the present invention to various organs with high specificity and high efficiency.

A person skilled in the art can produce, by selecting a known method suitably, a first nucleic acid strand and a second nucleic acid strand that constitute a nucleic acid complex according to various embodiments of the present invention. For example, nucleic acids according to some of the embodiments of the present invention can be produced by designing each nucleotide sequence of the nucleic acid based on information of the nucleotide sequence of a target miRNA, synthesizing a nucleic acid using a commercially available automated nucleic acid synthesis device (a product of Thermo Fisher Scientific, Inc., a product of Beckman Coulter, Inc., or the like), and then purifying the resulting oligonucleotide using a reversed phase column and the like. A nucleic acid produced by this method is mixed in a suitable buffer solution and denatured at about 90°C to 98°C for several minutes (for example, five minutes), the nucleic acid is then annealed at about 30°C to 70°C for about one to eight hours, and thus, a nucleic acid complex according to some of the embodiments of the present invention can be produced. Preparation of an annealed nucleic acid complex is not limited to such a time and temperature protocol. Conditions suitable to promote annealing of strands are well known in the art. A nucleic acid complex further linked to a functional moiety can be produced by using the kind of nucleic acid that has a functional moiety linked thereto in advance and carrying out the above-mentioned synthesis, purification, and annealing. Many methods for linking a functional moiety to a nucleic acid are well known in the art. Alternatively, a nucleic acid strand according to some of the embodiments can be ordered and obtained from a manufacturer (for example, GeneDesign Inc.), while specifying the nucleotide sequence and the site and type of modification.

The nucleic acid complex according to some embodiments is efficiently delivered to a living body (particularly, the liver), as shown in Examples mentioned later, and can effectively suppress a target miRNA level and inhibit a function of the target miRNA (disinhibit the expression of a target gene under the control of the target miRNA). Thus, the nucleic acid complex according to some embodiments can be used for suppressing a target miRNA level or inhibiting a function of the target miRNA.

The present applicant has reported a double-stranded antisense nucleic acid having an exon skipping effect (see International Publication No. WO 2014/203518). In general, pre-messenger RNA (mRNA) having exons and introns results from gene transcription. The introns are removed from the pre-mRNA (splicing) in the cellular nucleus to generate mature mRNA, and subsequently it is translated into a protein. The double-stranded antisense nucleic acid having an exon skipping effect can provide an enhanced exon skipping effect through enhanced delivery into the cellular nucleus (see International Publication No. WO 2014/203518). Meanwhile, the present invention relates to a double-stranded nucleic acid complex targeting miRNA present in the cytoplasm, not in the cellular nucleus. Accordingly, the double-stranded antisense nucleic acid having an exon skipping effect and the double-stranded nucleic acid complex according to the present invention differ in their targets and further in intracellular sites on which they act.

### <Composition and treatment and/or prevention method>

The present invention also provides a composition for suppressing a target miRNA level or inhibiting a function of target miRNA, comprising the nucleic acid complex described above as an active ingredient. The composition may be a pharmaceutical composition. As used herein, the term "target miRNA level" is used interchangeably with a "target miRNA expression level".

The compositions comprising the nucleic acid complex according to some embodiments of the present invention can be formulated using a known pharmaceutical manufacturing method. For example, the present composition can be used orally or parenterally in the form of capsules, tablets, pills, liquid, powder, granules, microgranules, film coated formulations, pellets, troches, sublingual formulations, peptizers, buccals, pastes, syrups, suspensions, elixirs, emulsions, coating agents, ointments, plasters, cataplasms, transdermal formulations, lotions, inhalants, aerosols, eyedrops, injection solutions, and suppositories.

With regard to formulating these formulations, pharmacologically acceptable carriers or carriers acceptable as food and beverage can be suitably incorporated, specific examples thereof including sterile water, physiological saline, plant oil, solvents, bases, emulsifying agents, suspending agents, surfactants, pH adjustors, stabilizers, flavoring agents, perfumes, excipients, vehicles, antiseptics, binders, diluents, isotonizing agents, sedatives, expanders, disintegrators, buffers, coating agents, lubricants, coloring agents, sweetners, thickeners, flavoring substances, dissolving auxiliaries, and other additives.

Forms of administration of the composition are not particularly limited, and examples thereof include oral administration or parenteral administration, more specifically, intravenous administration, intraventricular adiministration, intrathecal administration, subcutaneous administration, intraarterial administration, intraperitoneal administration, intradermal administration, tracheal bronchial administration, rectal administration, intraocular administration, and intramuscular administration, and administration by transfusion etc.

The composition can be used for animals, including humans, as subjects. However, animals other than humans are not limited to particular animals, and various animals such as farm animals, poultry, pet animals, and laboratory animals may be subjects in some embodiments.

Examples of the disease to be treated with the pharmaceutical composition include central nervous system diseases, metabolic diseases, tumors, and infections. Examples of the central nervous system diseases include, but are not particularly limited to, brain tumor, Alzheimer's disease, Parkinson's disease, Huntington's disease, corticobasal degeneration, progressive supranuclear palsy, Dementia with Lewy Bodies, Pick's disease, and amyotrophic lateral sclerosis.

When administering or taking the composition, the dose or the intake can be properly selected depending on the age, body weight, symptoms and health condition of a subject, the type of the composition (a medicament, a food and a beverage, etc.), etc. For example, the effective intake of the composition can be 0.0000001 mg/kg/day to 1000000 mg/kg/day, 0.00001 mg/kg/day to 10000 mg/kg/day or 0.001 mg/kg/day to 100 mg/kg/day of the nucleic acid complex.

The present invention also provides a method for suppressing a target miRNA level, comprising administering the nucleic acid complex or the composition of some embodiments to a subject in need thereof.

The present invention also provides a method for inhibiting a function of a target miRNA, comprising administering the nucleic acid complex or the composition of some embodiments to a subject in need thereof.

The present invention also provides a method for treating or preventing a disease related to increase in a target miRNA level, comprising administering the nucleic acid complex or the composition of some embodiments to a subject in need thereof.

The present invention also provides a method for treating or preventing hepatitis C virus infection, comprising administering the nucleic acid complex or the composition inhibiting a function of miR-122 according to some embodiments to a subject in need thereof. As mentioned above, miR-122 is miRNA highly expressed in the liver and is important for the stability and proliferation of hepatitis C virus (HCV) RNA. The treatment of hepatitis C virus infection can involve decreasing an HCV RNA level. Those skilled in the art can readily determine the HCV RNA level by use of a technique such as quantitative RT-PCR. The hepatitis C virus can be genotype 1.

The present invention also provides a method for treating or preventing a disease, comprising administering the nucleic acid complex or the composition inhibiting a function of miR-21 according to some embodiments to a subject in need thereof. Examples of the target disease of the present invention include cancers, Alport's syndrome, myocardial hypertrophy, cardiac fibrosis, and systemic lupus erythematosus (SLE). Examples of the cancers include hepatocellular cancer, lung cancer, ovary cancer, head and neck cancer, colorectal cancer, lung cancer, hepatocellular cancer, brain tumor, esophageal cancer, prostate cancer, pancreatic cancer, and thyroid gland cancer etc.

### Examples

Hereinafter, the present invention will be described further specifically with reference to Examples. However, the technical scope of the present invention is not limited by these Examples.

The sequences of the oligonucleotides used in Examples 1 to 10 given below are summarized in Table 1. All the oligonucleotides were synthesized by GeneDesign, Inc. (Osaka, Japan) under a commission.

**[Table 1]**

| Oligonucleotide name | Sequence (5'-3') | SEQ ID NO | Example |
|---|---|---|---|
| LNA/DNA antimiR-122 | c*c*a*t*t*g*t*c*a*c*a*c*t*c*c | 4 | 1-5, 7-10 |
| antimiR-122 cRNA (6OM 6PS) | G*G*A*GUGUGACAA*U*G*G | 5 | 1-5, 7-10 |
| Toc-LNA/DNA antimiR-122 | Toc-c*c*a*t*t*g*t*c*a*c*a*c*t*c*c | 4 | 7 |
| Toc-antimiR-122 cRNA (6OM 6PS) | Toc-G*G*A*GUGUGACAA*U*G*G | 5 | 7 |
| antimiR-122 cRNA (0OM 0PS) | GGAGUGUGACAAUGG | 5 | 8 |
| antimiR-122 cRNA(6OM 0PS) | GGAGUGUGACAAUGG | 5 | 8 |
| antimiR-122 cRNA(0OM6PS) | G'G'A'GUGUGACAA'U'G'G | 5 | 8 |
| antimiR-122 cRNA (6OM 14PS) | G*G*A*G*U*G*U*G*A*C*A*A*U*G*G | 5 | 9 |
| antimiR-122 cRNA (2OM 2PS) | G'GAGUGUGACAAUG'G | 5 | 10 |
| antimiR-122 cRNA (10OM 10PS) | G*G*A*G*U*GUGAC*A*A*U*G*G | 5 | 10 |
| LNA/DNA antimiR-21 | t*c*a*g*t*c*t*g*a*t*a*a*g*c*t | 6 | 6 |
| antimiR-21 cRNA (6OM 6PS) | A*G*C*UUAUCAGAC*U*G*A | 7 | 6 |

| | | | |
|---|---|---|---|
| Underlined lower-case character: LNA (c represents 5-methylcytosine LNA) Lower-case character: DNA Upper-case character: RNA Underlined upper-case character: 2'-O-methyl RNA *: phosphorothioate linkage Toc: tocopherol | | | |

### [Example 1]

### (miR-122 suppressive effect of double-stranded nucleic acid complex targeting miR-122)

An *in vivo* experiment was conducted to verify the usefulness of the double-stranded nucleic acid agent according to one embodiment. Specifically, the microRNA suppressive effect of heteroduplex oligonucleotide-antimiR (hereinafter, referred to as "HDO-antimiR"), a double-stranded agent of one embodiment, was evaluated by using a conventional single-stranded LNA/DNA mixmer type microRNA suppressive drug (hereinafter, referred to as "antimiR") as a control.

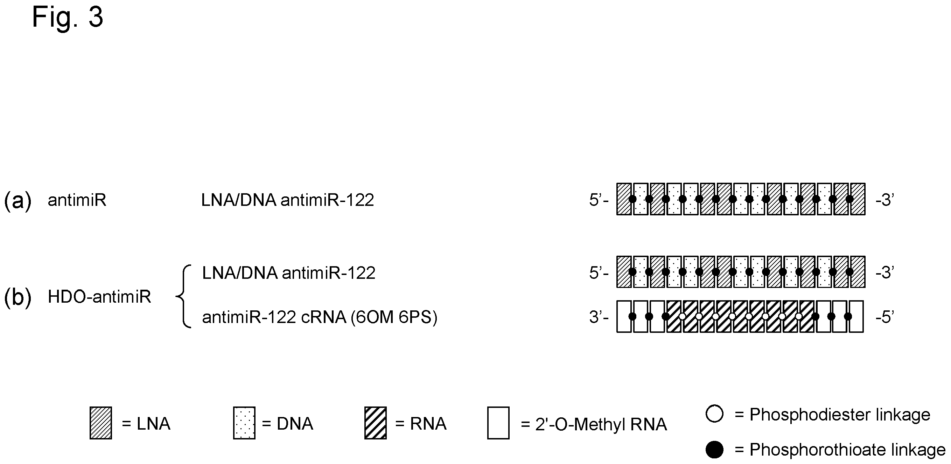

The antimiR used as a control in Example 1 was a 15-mer LNA/DNA mixmer (oligonucleotide name: LNA/DNA antimiR-122) complementary to positions 2 to 16 of mouse microRNA-122 (miR-122) (SEQ ID NO: 1). The nucleosides constituting this LNA/DNA mixmer were eight LNA nucleosides and seven natural deoxyribonucleosides. This LNA/DNA mixmer is an oligonucleotide formed from these nucleosides bound via phosphorothioate linkages. This LNA/DNA mixmer has up to two consecutive LNA nucleosides and up to two consecutive DNA nucleosides (Figure 3a).

The double-stranded agent "HDO-antimiR" has the LNA/DNA mixmer (first nucleic acid strand) described above, and a second nucleic acid strand completely complementary to the first nucleic acid strand (complementary strand annealing with the first nucleic acid strand; oligonucleotide name: antimiR-122 cRNA (6OM 6PS)) and forms a double-stranded structure (Figure 3b). The nucleosides constituting the second nucleic acid strand were 5'-terminal three 2'-O-methyl ribonucleosides, 3'-terminal three 2'-O-methyl ribonucleosides, and nine natural ribonucleosides flanked thereby. The internucleoside linkages of the second nucleic acid strand were 5'-terminal three phosphorothioate linkages, 3'-terminal three phosphorothioate linkages, and phosphodiester linkages at sites other than their sites.

The sequences, chemical modifications and structures of the oligonucleotides used in Example 1 are shown in Table 1 and Figure 3.

A solution containing the first nucleic acid strand and the second nucleic acid strand mixed in equimolar amounts was heated at 95°C for 5 minutes, then cooled to 37°C, and kept for 1 hour so that these nucleic acid strands were annealed with each other to prepare the double-stranded agent (double-stranded nucleic acid complex) described above. The annealed nucleic acid was preserved at 4°C or on ice.

Four-week-old female ICR mice (Charles River Laboratories Japan, Inc.) having a body weight of 20 to 25 g were used. Each nucleic acid agent was intravenously injected at a dose of 23.56 nmol/kg to the mice (n = 5) through their tail veins. Further, mice to which PBS alone (instead of the nucleic acid agent) was injected were also prepared as a negative control group. 72 hours after the injection, the mice were perfused with PBS. Then, the mice were dissected to harvest their livers.

Total RNA comprising microRNA was extracted from the harvested livers using MagNA Pure 96 Cellular RNA Large Volume Kit (F. Hoffmann-La Roche, Ltd.) and MagNA Pure 96 system (F. Hoffmann-La Roche, Ltd.) according to the protocol of the system. cDNA was synthesized from the RNA using TaqMan MicroRNA Assays (Thermo Fisher Scientific Inc.) according to the protocol. Quantitative RT-PCR was performed using the synthesized cDNA. The primers used in the quantitative RT-PCR were products designed and manufactured by Thermo Fisher Scientific Inc. (formerly, Life Technologies Corp) on the basis of various gene numbers. The expression level of miR-122 was divided by the expression level of U6 (internal standard gene) on the basis of the results of the quantitative RT-PCR thus obtained. A mean and standard deviation of each group were calculated as to the obtained value. The relative miR-122 level of each group was calculated such that the mean of the PBS administration group was 1. The results about the groups were compared and further evaluated by the Bonferroni test.

### (Results)

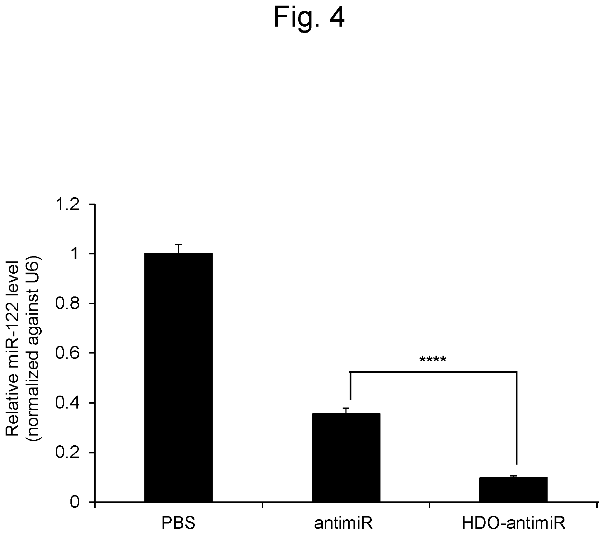

The results of Example 1 are shown in the graph of Figure 4. The single-stranded antimiR and the double-stranded agent HDO-antimiR suppressed the miR-122 level as compared with the negative control (PBS alone). The double-stranded HDO-antimiR according to one embodiment of the present invention statistically significantly suppressed the miR-122 level as compared with the single-stranded antimiR.

These results indicated that the double-stranded nucleic acid complex according to one embodiment of the present invention provides a high antisense effect (suppression of the target miRNA level) as compared with the conventional single-stranded mixmer type antisense nucleic acid.

### [Example 2]

### (Effective dose 50% analysis)

The double-stranded nucleic acid agent according to one embodiment was evaluated for its dose achieving 50% suppression of microRNA (effective dose 50% (ED50)). The double-stranded HDO-antimiR used in Example 1 was compared with the single-stranded antimiR for evaluation.

The single-stranded antimiR used in Example 1 was administered at a dose of 5.9, 23.6, 58.9 or 117.8 nmol/kg to mice (n = 5) through their tail veins. The double-stranded HDO-antimiR used in Example 1 was administered at a dose of 0.04, 0.59, 5.9, or 23.6 nmol/kg to mice (n = 5) through their tail veins. Mice to which PBS alone (instead of the nucleic acid agent) was injected were also prepared as a negative control group.

Relative miR-122 levels in the livers were calculated by mouse extracting liver, extracting RNA from the livers, synthesizing cDNA and quantitative RT-PCR as described in Example 1. The ED50 value of each nucleic acid agent was calculated from the doses and the values of the relative miR-122 levels using Prism version 6.05 (GraphPad Software).

### (Results)

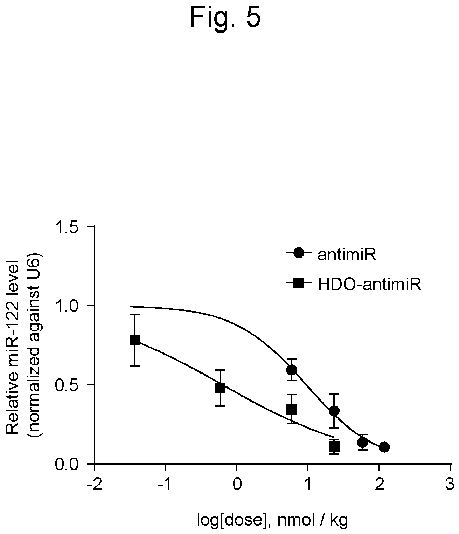

The relationship between the doses and the relative miR-122 levels is shown in the graph of Figure 5. The ED50 value of the single-stranded antimiR was 9.46 nmol/kg, whereas the ED50 value of the double-stranded HDO-antimiR was 0.63 nmol/kg, indicating that the double-stranded HDO-antimiR exhibited approximately 15-fold improvement in suppressive effect as compared with the single-stranded antimiR. These results indicated that the double-stranded nucleic acid complex according to one embodiment of the present invention can very strongly suppress the target miRNA in a concentration-dependent manner as compared with the conventional single-stranded mixmer type antisense nucleic acid.

### [Example 3]

### (Evaluation of ability to bind to target miRNA)

An experiment was conducted to verify a mechanism underlying the improved miRNA suppressive effect of the double-stranded nucleic acid agent according to one embodiment. The conventional single-stranded agent serving as a miRNA suppressive drug is known to bind directly to mature miRNA, which is a final product after the processing process of miRNA, and inhibit a function thereof. The ability of the double-stranded agent to bind to a mature target miRNA was evaluated through comparison with the single-stranded agent by Northern blotting using a probe against the mature miRNA.

As described in Example 1, the single-stranded agent antimiR or the double-stranded agent HDO-antimiR was administered to mice, and the livers were harvested from the mice, followed by RNA extraction from the livers. Total RNA (30 µg) from the single-stranded agent administration group or the double-stranded agent administration group was loaded in each lane and separated by electrophoresis on 18% polyacrylamide/urea gel. Chemically synthesized mature miR-122, and a duplex prepared by the preliminary heating and cooling treatments of mature miR-122 and the antimiR used in Example 1 were included as size markers in other lanes. The gel after the electrophoresis was transferred to Hybond-N+ membrane (GE Healthcare Japan Corp. (formerly, Amersham Biosciences Corp.)). A probe recognizing mature miR-122 (miRCURY LNA Detection Probe, Exiqon) or a probe recognizing internal standard U6 small molecule RNA (5'-TGGTGCGTATGCGTAGCATTGGTATTCA-3', SEQ ID NO: 3) was hybridized to the membrane and visualized using Gene Images CDP-star Detection Kit (GE Healthcare Japan Corp.).

### (Results)

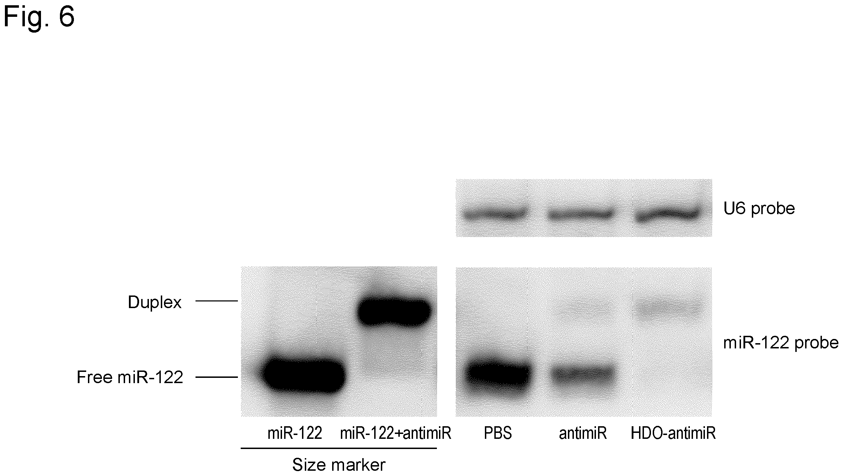

The results of Example 3 are shown in Figure 6. In the RNA samples obtained from the single-stranded antimiR administration group, many molecules of mature miR-122 remained as single strands while only some molecules of mature miR-122 bound to the antimiR. On the other hand, in the RNA samples obtained from the double-stranded HDO-antimiR administration group, almost all the molecules of mature miR-122 were found to bind to the antisense strands to form duplexes.

These results indicated that the double-stranded HDO-antimiR has high ability to bind to mature miRNA, which is a final product of the processing process of miRNA, as compared with the single-stranded antimiR. This improvement in the ability to bind was found to provide improvement in the suppression of the target miRNA by the double-stranded nucleic acid complex according to one embodiment of the present invention.

### [Example 4]

### (Disinhibitory effect of double-stranded nucleic acid complex targeting miR-122 on downstream target gene of miR-122)

miR-122 suppressively controls the mRNA expression of aldolase A (ALDOA) and branched chain ketoacid dehydrogenase kinase (BCKDK) (Elmen J et al., LNA-mediated microRNA silencing in non-human primates. Nature, 2008, 452 (7189): 896-899). The HDO-antimiR targeting miR-122, used in Example 1 binds to miR-122 and inhibits a function thereof, presumably resulting in increase in ALDOA and BCKDK mRNA expression levels (i.e., disinhibition). The HDO-antimiR targeting miR-122, used in Example 1 was evaluated for its disinhibitory effect on ALDOA and BCKDK mRNA expression levels. A total serum cholesterol value correlating with the ALDOA expression level was also evaluated (Elmen J et al., supra).

The control single-stranded agent antimiR and the double-stranded agent HDO-antimiR used in Example 1 were used. The double-stranded agent was prepared in the same way as in Example 1.

Each nucleic acid agent was intravenously injected at a dose of 0.14 or 0.35 µmol/kg to mice (n = 5) through their tail veins. The injection was performed three times a week (on days 0, 3 and 7). Mice to which PBS alone (instead of the nucleic acid agent) was injected were also prepared as a negative control group. 168 hours after the final injection, the mice were perfused with PBS. Then, the mice were dissected to harvest their livers. Blood was collected at the time of each injection, 168 hours after the final injection, and upon harvesting of the liver tissues.

As described in Example 1, RNA was extracted from the harvested livers, and cDNA was synthesized from the RNA, followed by quantitative RT-PCR using the synthesized cDNA. However, in the quantitative RT-PCR, ALDOA, BCKDK, and internal standard gene βActin (ActB) mRNA levels were determined. On the basis of the obtained results of the quantitative RT-PCR, the ALDOA mRNA expression level was divided by the βActin mRNA expression level, and the BCKDK mRNA expression level was divided by the βActin mRNA expression level. A mean and standard deviation were calculated as to the obtained values. The relative ALDOA mRNA level and the relative BCKDK mRNA level of each group were calculated such that the mean of the PBS administration group was 1. The results about the groups were compared and further evaluated by the Bonferroni test.

Percent decrease (%) in total serum cholesterol was calculated by subtracting the total serum cholesterol value obtained 168 hours after the final injection from the total serum cholesterol value obtained 0 hours after the initial injection (at the time of the initial injection), dividing the obtained value by the total serum cholesterol value obtained 0 hours after the initial injection (at the time of the initial injection), and multiplying the obtained value by 100. The mean and standard deviation of each group were calculated. The results about the groups were compared and further evaluated by the Bonferroni test.

### (Results)

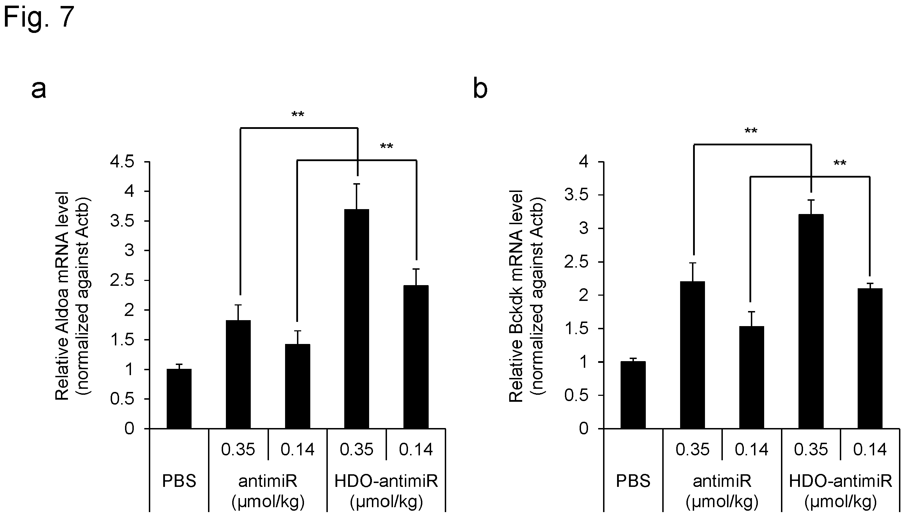

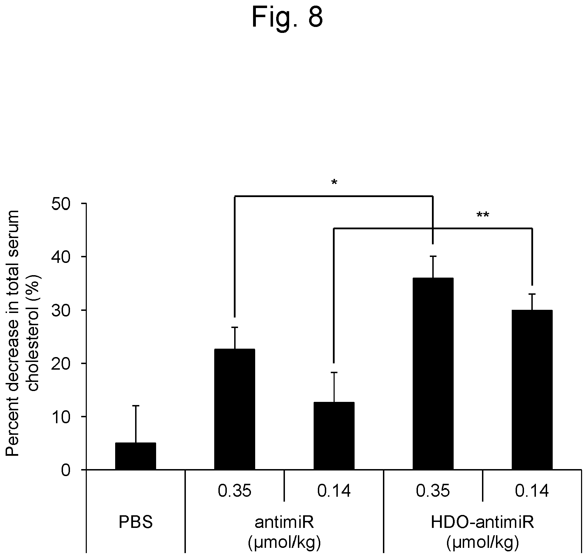

The results of Example 4 are shown in the graphs of Figures 7 and 8. The double-stranded agent HDO-antimiR exhibited statistically significant increase in the expression level of the downstream target (ALDOA and BCKDK) mRNA of miR-122 (i.e., disinhibition of the downstream target gene) as compared with the single-stranded antimiR (Figures 7a and 7b). The degree of the increase in the expression level of the downstream target (ALDOA and BCKDK) mRNA of miR-122 was larger for the dose of 0.35 µmol/kg than for the dose of 0.14 µmol/kg.

The double-stranded agent HDO-antimiR exhibited statistically significant decrease in total serum cholesterol (correlating with the ALDOA expression level) as compared with the single-stranded antimiR (Figure 8). The degree of the decrease in total serum cholesterol was larger for the dose of 0.35 µmol/kg than for the dose of 0.14 µmol/kg.

These results indicated that the double-stranded nucleic acid complex according to one embodiment of the present invention exhibits a disinhibitory effect on the downstream target gene of miRNA in a dose-dependent manner, and the effect is higher than that of the single-stranded antimiR.

### [Example 5]

### (Evaluation of hepatotoxicity and nephrotoxicity of double-stranded nucleic acid complex)

The double-stranded nucleic acid agent according to one embodiment was evaluated for its hepatotoxicity and nephrotoxicity.

As described in Example 4, each nucleic acid agent was administered to mice, and blood was collected. AST (aspartate aminotransferase) and ALT (alanine aminotransferase) activity values, and total bilirubin in serum obtained 168 hours after the final injection were measured as indices for hepatotoxicity. BUN (blood urea nitrogen) and creatinine values in serum obtained 168 hours after the final injection were measured as indices for nephrotoxicity.

### (Results)

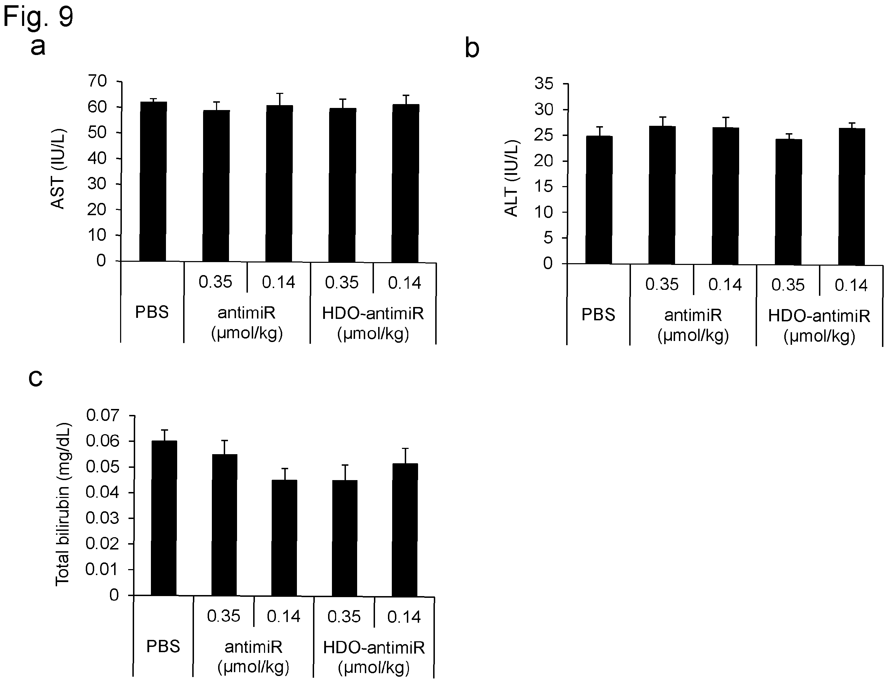

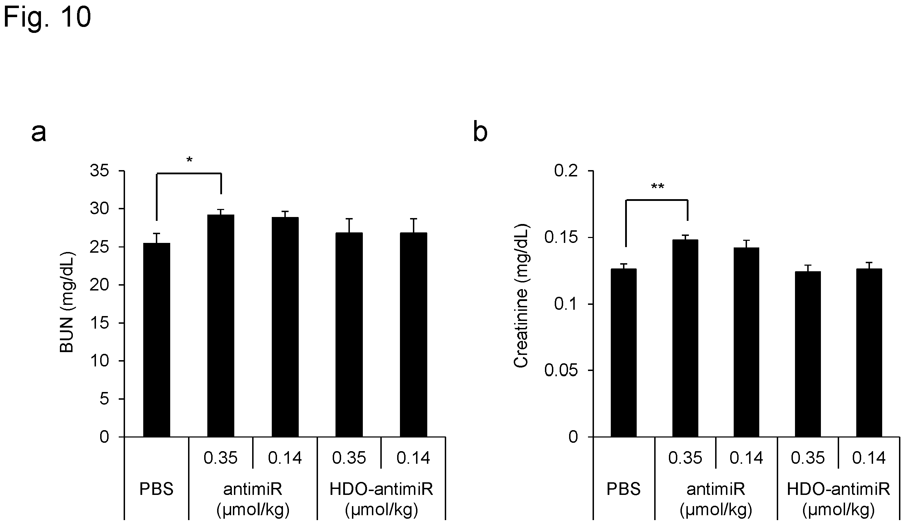

The results of Example 5 are shown in the graphs of Figures 9 and 10. Both the single-stranded antimiR and the double-stranded HDO-antimiR neither influenced AST, ALT and total bilirubin nor exhibited hepatotoxicity (Figures 9a to 9c).

On the other hand, in the group given 0.35 µmol/kg of the single-stranded antimiR, BUN and creatinine in serum were significantly elevated as compared with the PBS group, indicating nephrotoxicity. However, in the group given the double-stranded HDO-antimiR, BUN and creatinine in serum were not elevated for both the doses of 0.35 µmol/kg and 0.14 µmol/kg (Figures 10a and 10b). This indicates that the double-stranded nucleic acid complex according to one embodiment of the present invention ameliorates nephrotoxicity caused by the single-stranded antimiR.

### [Example 6]

### (Disinhibitory effect of double-stranded nucleic acid complex targeting miR-21 on downstream target gene of miR-21)

An *in vivo* experiment was conducted to verify the usefulness of the double-stranded nucleic acid agent according to one embodiment targeting microRNA-21 (miR-21), which is microRNA different from miR-122 targeted in Examples 1 to 5. Specifically, the double-stranded nucleic acid agent was evaluated for its disinhibitory effect on the mRNA expression of downstream target genes Spg20 (spastic paraplegia 20) and Taf7 (TATA-box binding protein associated factor 7) of miR-21.

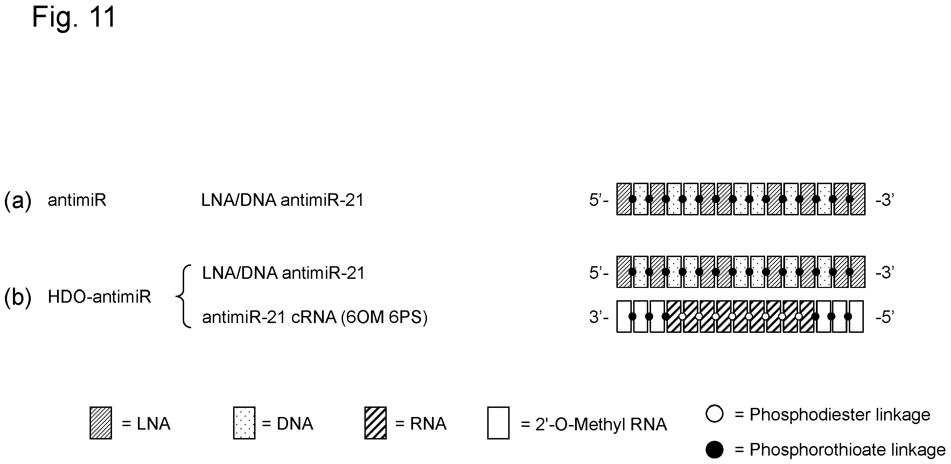

The antimiR used as a control in Example 6 was a 15-mer LNA/DNA mixmer (oligonucleotide name: LNA/DNA antimiR-21) complementary to positions 2 to 16 of mouse microRNA-21 (miR-21) (SEQ ID NO: 2). The nucleosides constituting this LNA/DNA mixmer were eight LNA nucleosides and seven natural deoxyribonucleosides. This LNA/DNA mixmer is an oligonucleotide formed from these nucleosides linked via phosphorothioate linkages. This LNA/DNA mixmer has up to two consecutive LNA nucleosides and up to two consecutive DNA nucleosides (Figure 11a).

The double-stranded agent "HDO-antimiR" consists of the LNA/DNA mixmer (first nucleic acid strand) described above, and a second nucleic acid strand completely complementary to the first nucleic acid strand (complementary strand annealing with the first nucleic acid strand; oligonucleotide name: antimiR-21 cRNA (6OM 6PS)) and forms a double-stranded structure (Figure 11b). The nucleosides constituting the second nucleic acid strand were 5'-terminal three 2'-O-methyl ribonucleosides, 3'-terminal three 2'-O-methyl ribonucleosides, and nine natural ribonucleosides flanked thereby. The internucleoside linkages of the second nucleic acid strand were 5'-terminal three phosphorothioate linkages, 3'-terminal three phosphorothioate linkages, and phosphodiester linkages at sites other than their sites.

The sequences, chemical modifications and structures of the oligonucleotides used in Example 6 are shown in Table 1 and Figure 11. The double-stranded agent was prepared in the same way as in Example 1.

Each nucleic acid agent was intravenously injected at a dose of 5 or 20 nmol/kg to mice (n = 5) through their tail veins. Mouse liver harvesting, RNA extraction from the livers, cDNA synthesis and quantitative RT-PCR were performed as described in Example 1. However, in the quantitative RT-PCR, Spg20, Taf7, and internal standard gene βActin (ActB) mRNA levels were determined. On the basis of the obtained results of the quantitative RT-PCR, the Spg20 mRNA expression level was divided by the βActin mRNA expression level, and the Taf7 mRNA expression level was divided by the βActin mRNA expression level. A mean and standard deviation were calculated as to the obtained values. The relative Spg20 mRNA level and the relative Taf7 mRNA level of each group were calculated such that the mean of the PBS administration group was 1. The results about the groups were compared and further evaluated by the Bonferroni test.

### (Results)

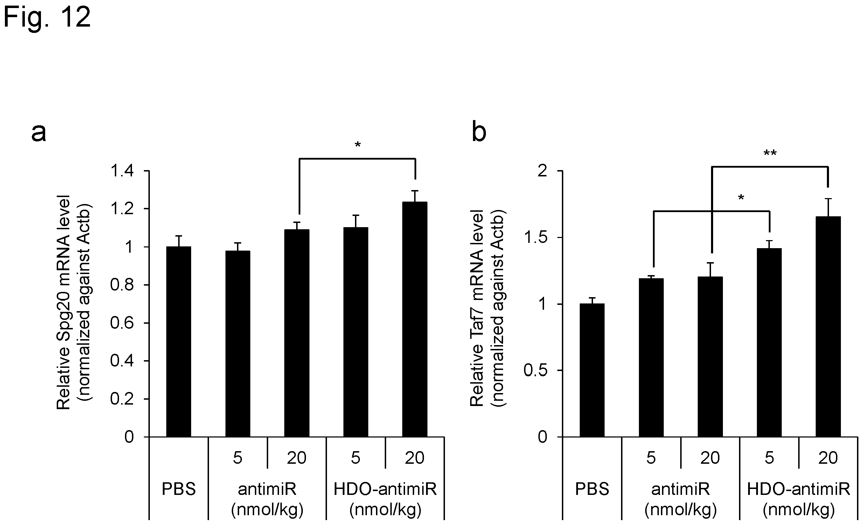

The results of Example 6 are shown in the graph of Figure 12. The double-stranded agent HDO-antimiR exhibited statistically significant increase in the expression level of the downstream target (Spg20 and Taf7) mRNA of miR-21 (i.e., disinhibition of the downstream target gene) as compared with the single-stranded antimiR (Figure 12). The degree of the increase in the expression level of the downstream target (Spg20 and Taf7) mRNA of miR-21 was larger for the dose of 20 nmol/kg than for the dose of 5 nmol/kg.

These results indicated that the effect of the double-stranded nucleic acid complex according to one embodiment of the present invention is not specific for miR-122, and the double-stranded nucleic acid complex is capable of targeting various miRNAs. These results also indicated that the double-stranded nucleic acid complex exhibits a disinhibitory effect on the downstream target gene of miRNA in a dose-dependent manner, and the effect is higher than that of the single-stranded antimiR.

### [Example 7]

### (Double-stranded nucleic acid complex bound with ligand molecule)

An *in vivo* experiment was conducted to verify the usefulness of the miR-122-targeting double-stranded nucleic acid agent according to one embodiment bounded with a vitamin E ligand molecule (α-tocopherol: Toc). It is known that a nucleic acid agent bound with α-tocopherol is efficiently delivered to the liver (see, for example, International Publication No. WO 2013/089283).

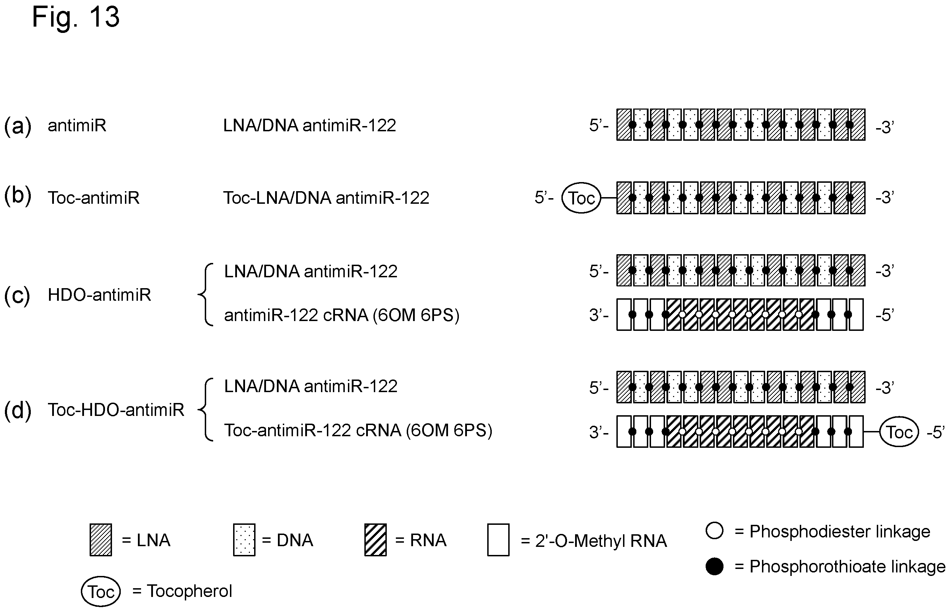

Controls were the conventional single-stranded agent targeting miR-122 (antimiR), used in Example 1, and a single-stranded agent of antimiR bound with α-tocopherol at 5'-terminal (Toc-antimiR) (Figures 13a and 13b). The double-stranded agent (HDO-antimiR, Figure 13c) used in Example 1, and a double-stranded agent having the second nucleic acid strand (strand complementary to the LNA/DNA mixmer serving as the first nucleic acid strand) bound with α-tocopherol at 5'-terminal (Toc-HDO-antimiR, Figure 13d) were used and evaluated for their miR-122 suppressive effect. The sequences, chemical modifications and structures of the oligonucleotides used in Example 7 are shown in Table 1 and Figure 13. The double-stranded agents were prepared in the same way as in Example 1.

Each nucleic acid agent was intravenously injected at a dose of 0.05 µmol/kg to mice (n = 4) through their tail veins. The mice and the miR-122 expression analysis method used were the same as those of Example 1.

### (Results)

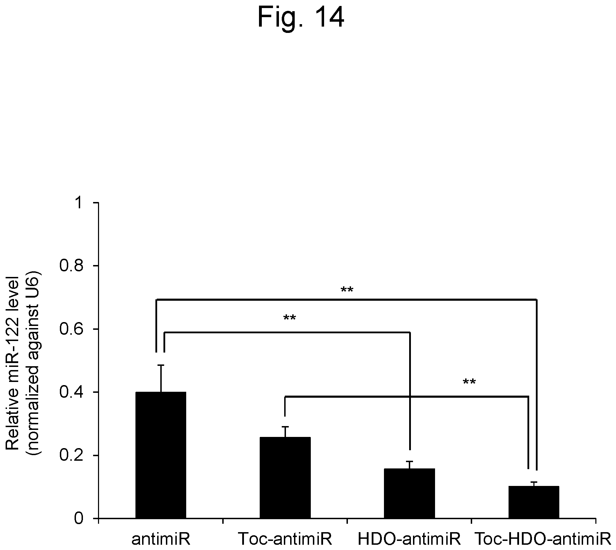

The results of Example 7 are shown in the graph of Figure 14. The tocopherol-bound double-stranded agent Toc-HDO-antimiR exhibited statistically significant suppression of miR-122 expression as compared with the single-stranded antimiR and Toc-antimiR. The tocopherol-bound double-stranded agent Toc-HDO-antimiR exhibited a tendency to suppress the expression of miR-122 as compared with the tocopherol-free double-stranded agent HDO-antimiR. These results indicated that the binding of the ligand molecule to the double-stranded nucleic acid complex according to one embodiment of the present invention increases the suppressive effect on the target miRNA.

### [Example 8]

### (Evaluation of effect of chemical modification on activity of double-stranded nucleic acid complex)

An *in vivo* experiment was conducted to verify the usefulness of the double-stranded nucleic acid agent according to one embodiment comprising the second nucleic acid strand having a different chemical modification.

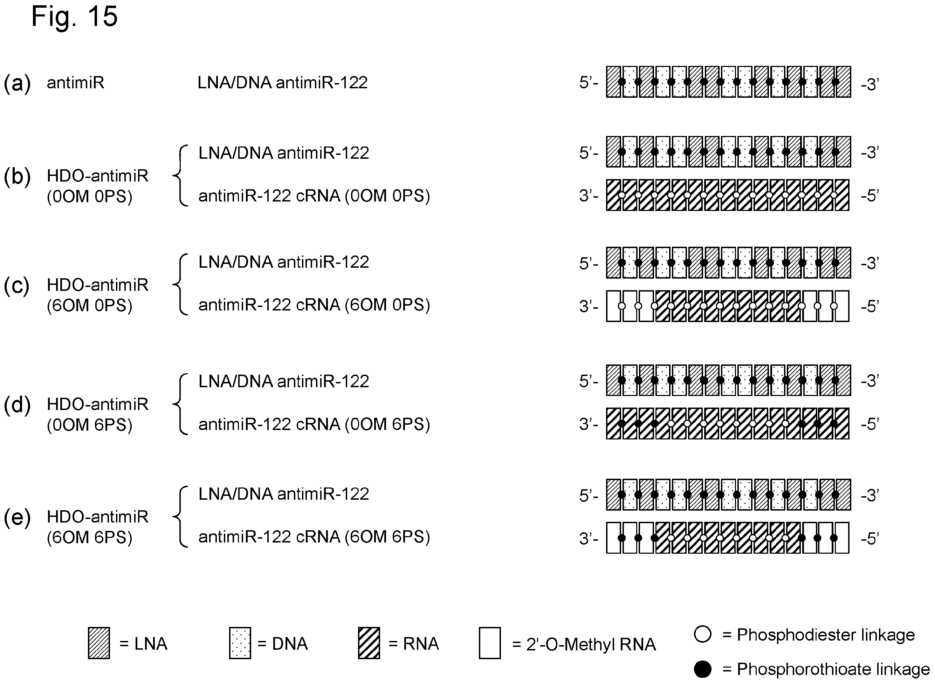

A control was the conventional single-stranded LNA/DNA mixmer targeting miR-122 (antimiR), used in Example 1 (Figure 15a). Four types of double-stranded agents consisting of this LNA/DNA mixmer (first nucleic acid strand) and variously chemically modified second nucleic acid strands complementary to the first nucleic acid strand were evaluated for their miR-122 suppressive effect. The following four types of double-stranded agents were used:
a double-stranded agent comprising an unmodified second nucleic acid strand (natural RNA) ("HDO-antimiR (OOM 0PS)", Figure 15b);
a double-stranded agent comprising the second nucleic acid strand having natural RNA as well as 5'-terminal three 2'-O-methyl ribonucleosides and 3'-terminal three 2'-O-methyl ribonucleosides ("HDO-antimiR (6OM 0PS)", Figure 15c);
a double-stranded agent comprising the second nucleic acid strand having natural RNA as well as 5'-terminal three phosphorothioate linkages and 3'-terminal three phosphorothioate linkages ("HDO-antimiR (OOM 6PS)", Figure 15d); and
a double-stranded agent comprising the second nucleic acid strand having natural RNA as well as 5'-terminal three 2'-O-methyl ribonucleosides and three phosphorothioate linkages, and 3'-terminal three 2'-O-methyl ribonucleosides and three phosphorothioate linkages ("HDO-antimiR (6OM 6PS)", Figure 15e).

The sequences, chemical modifications and structures of the oligonucleotides used in Example 8 are shown in Table 1 and Figure 15. The double-stranded agents were prepared in the same way as in Example 1.

Each nucleic acid agent was intravenously injected at a dose of 0.24 µmol/kg to mice (n = 4) through their tail veins. The mice and the miR-122 expression analysis method used were the same as those of Example 1.

### (Results)

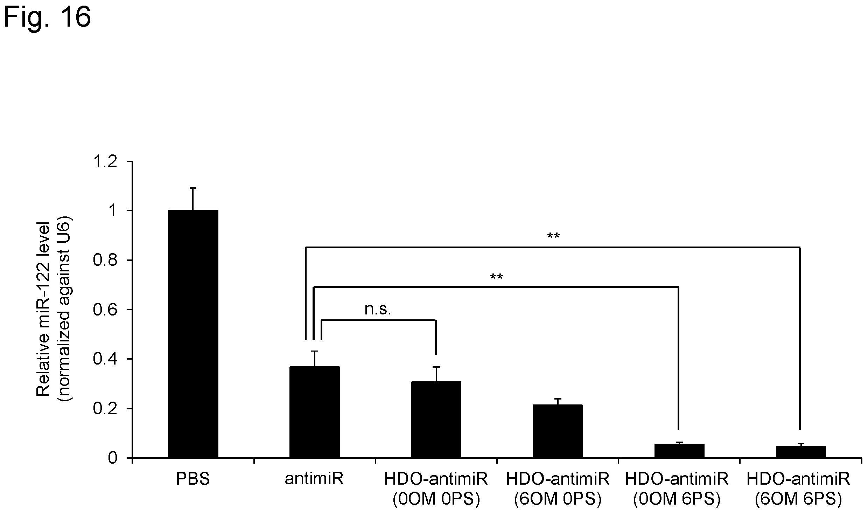

The results of Example 8 are shown in the graph of Figure 16. The double-stranded agent HDO-antimiR (6OM 0PS) exhibited a tendency to suppress the expression of miR-122 as compared with the single-stranded antimiR. The double-stranded agents HDO-antimiR (OOM 6PS) and HDO-antimiR (6OM 6PS) statistically significantly suppressed the expression of miR-122 as compared with the single-stranded antimiR. These results indicated that when the second nucleic acid strand of the double-stranded nucleic acid complex comprises modified internucleoside linkages (phosphorothioate linkages) and/or sugar modified (2'-0-methyl modified) nucleosides, the activity of suppressing microRNA is increased as compared with the single-stranded agent.

### [Example 9]

### (Further evaluation of effect of chemical modification on activity of double-stranded nucleic acid complex)

An *in vivo* experiment was further conducted to verify the usefulness of the double-stranded nucleic acid agent according to one embodiment comprising the second nucleic acid strand having a different chemical modification.

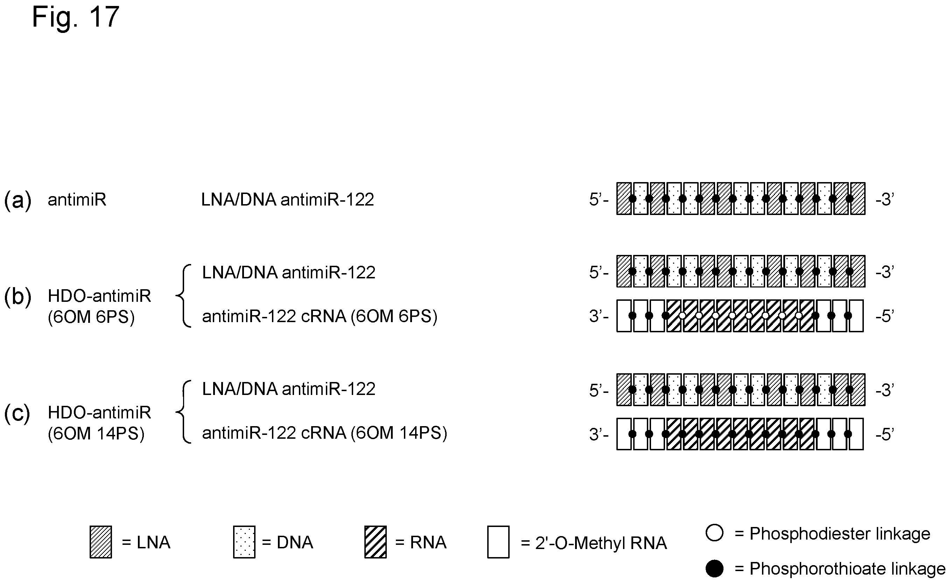

A control was the conventional single-stranded LNA/DNA mixmer targeting miR-122 (antimiR), used in Example 1 (Figure 17a). Two types of double-stranded agents consisting of this LNA/DNA mixmer (first nucleic acid strand) and variously chemically modified second nucleic acid strands complementary to the first nucleic acid strand were evaluated for their miR-122 suppressive effect. The following two types of double-stranded agents were used:
a double-stranded agent comprising the second nucleic acid strand having natural RNA as well as 5'-terminal three 2'-O-methyl ribonucleosides and three phosphorothioate linkages, and 3'-terminal three 2'-O-methyl ribonucleosides and three phosphorothioate linkages ("HDO-antimiR (6OM 6PS)", Figure 17b); and
a double-stranded agent comprising the second nucleic acid strand having natural RNA as well as 5'-terminal three 2'-O-methyl ribonucleosides and 3'-terminal three 2'-O-methyl ribonucleosides, and had phosphorothioate linkages as all the internucleoside linkages ("HDO-antimiR (6OM 14PS)", Figure 17c).

The sequences, chemical modifications and structures of the oligonucleotides used in Example 9 are shown in Table 1 and Figure 17. The double-stranded agents were prepared in the same way as in Example 1.

Each nucleic acid agent was intravenously injected at a dose of 0.24 µmol/kg to mice (n = 4) through their tail veins. The mice and the miR-122 expression analysis method used were the same as those of Example 1.

### (Results)

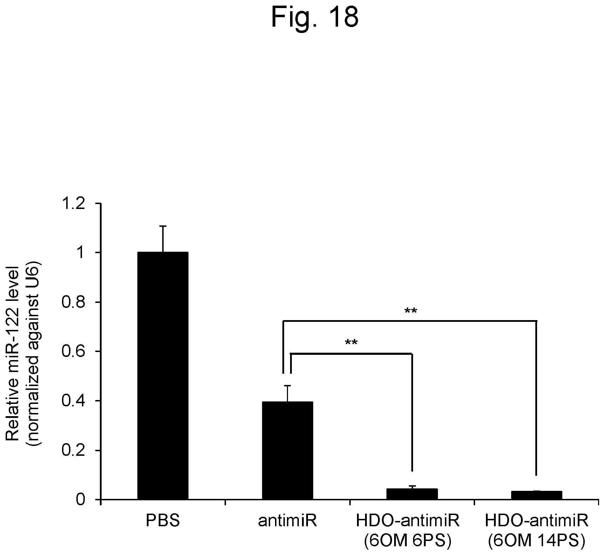

The results of Example 9 are shown in the graph of Figure 18. Both of the double-stranded agents HDO-antimiR (6OM 6PS) and HDO-antimiR (6OM 14PS) statistically significantly suppressed the expression of miR-122 as compared with the single-stranded antimiR. These results indicated that the double-stranded nucleic acid complex having phosphorothioate linkages as all the internucleoside linkages can efficiently suppress the target miRNA.

### [Example 10]

### (Evaluation of effect of length of consecutive natural ribonucleosides of second nucleic acid strand on activity of double-stranded nucleic acid complex)

An *in vivo* experiment was conducted to verify the usefulness of the double-stranded nucleic acid agent according to one embodiment comprising the second nucleic acid strand having a different length of consecutive natural ribonucleosides.

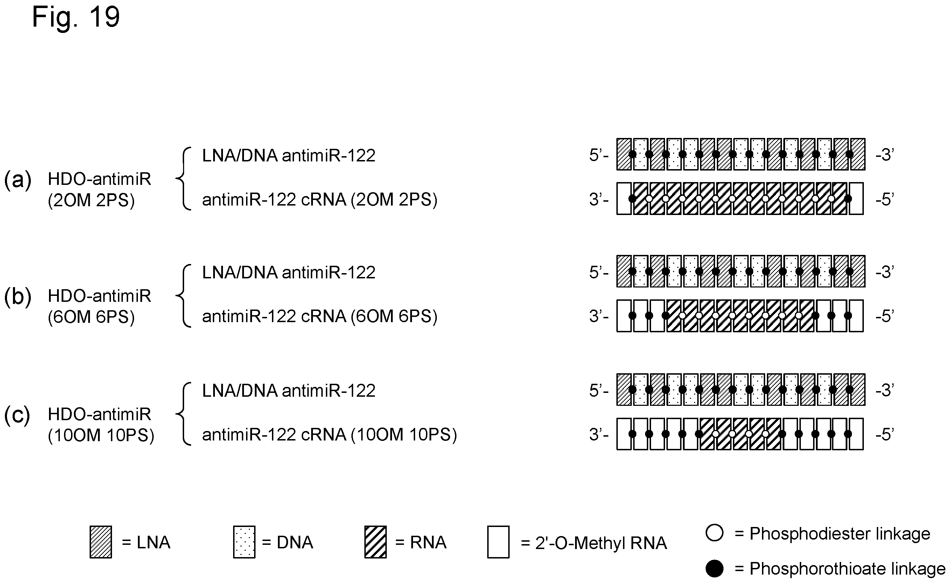

Three types of double-stranded agents consisting of the single-stranded LNA/DNA mixmer (first nucleic acid strand) 15 nucleotide length targeting miR-122, and the second nucleic acid strand 15 nucleotide length complementary to the first nucleic acid strand were evaluated for their miR-122 suppressive effect. The following three types of double-stranded agents were used:
a double-stranded agent comprising the second nucleic acid strand having natural RNA as well as 5'-terminal one 2'-O-methyl ribonucleoside and one phosphorothioate linkage, and 3'-terminal one 2'-O-methyl ribonucleoside and one phosphorothioate linkage (the second nucleic acid strand had consecutive natural ribonucleosides having a length of 13 bases) ("HDO-antimiR (2OM 2PS)", Figure 19a);
a double-stranded agent comprising the second nucleic acid strand having natural RNA as well as 5'-terminal three 2'-O-methyl ribonucleosides and three phosphorothioate linkages, and 3'-terminal three 2'-O-methyl ribonucleosides and three phosphorothioate linkages (the second nucleic acid strand had consecutive natural ribonucleosides having a length of 9 bases) ("HDO-antimiR (6OM 6PS)", Figure 19b); and
a double-stranded agent comprising the second nucleic acid strand having natural RNA as well as 5'-terminal five 2'-O-methyl ribonucleosides and five phosphorothioate linkages, and 3'-terminal five 2'-O-methyl ribonucleosides and five phosphorothioate linkages (the second nucleic acid strand had consecutive natural ribonucleosides having a length of 5 bases) ("HDO-antimiR (10OM 10PS)", Figure 19c).

The sequences, chemical modifications and structures of the oligonucleotides used in Example 10 are shown in Table 1 and Figure 19. The double-stranded agents were prepared in the same way as in Example 1.

Each nucleic acid agent was intravenously injected at a dose of 5.9 or 23.6 nmol/kg to mice (n = 4) through their tail veins. The mice and the miR-122 expression analysis method used were the same as those of Example 1.

### (Results)

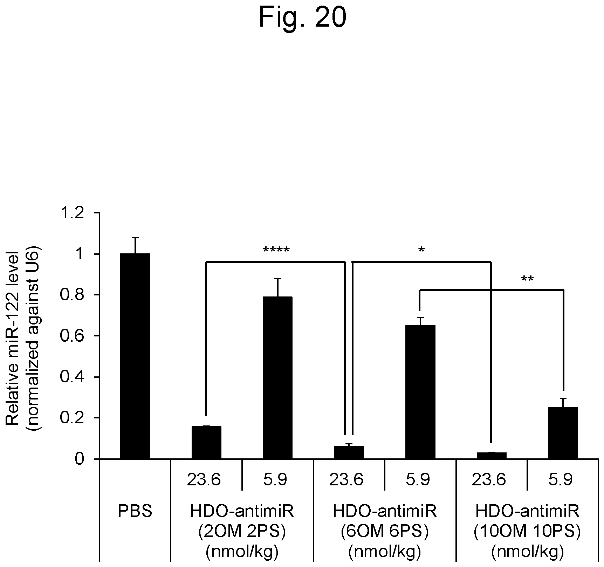

The results of Example 10 are shown in the graph of Figure 20. HDO-antimiR (10OM 10PS) most efficiently suppressed miR-122, and HDO-antimiR (6OM 6PS) and HDO-antimiR (2OM 2PS) also suppressed miR-122 (Figure 20). These results indicated that when the second nucleic acid strand has consecutive natural ribonucleosides having various lengths, the target miRNA is largely suppressed.

Particularly, miRNA suppressive activity was tend to increase as the length of consecutive natural ribonucleosides was decreased. The present applicant has previously stated that intracellular transcript levels are reduced using a double-stranded nucleic acid complex comprising a first nucleic acid strand that hybridizes to the transcript, and a second nucleic acid strand cleavable by RNaseH (see International Publication No. WO 2013/089283). On the basis of the previous findings, it is predicted that the presence of many (e.g., seven or more consecutive) natural ribonucleosides sensitive to degradation by RNase in the second nucleic acid strand is advantageous for the functioning of the double-stranded nucleic acid complex. However, as shown in the results of Example 10, the present invention indicated that 5 nucleotide length is sufficient for the length of consecutive natural ribonucleosides, and rather, the activity was tend to increase as the length of consecutive natural ribonucleosides is short. These results are unexpectable from the previous findings described in International Publication No. WO 2013/089283, etc.

### [Example 11]

The sequences of the oligonucleotides used in Examples 11 to 14 given below are summarized in Table 2. All the oligonucleotides were synthesized by GeneDesign, Inc. (Osaka, Japan) under a commission.

**[Table 2]**

| Oligonucleotide name | Sequence (5'-3') | SEQ ID NO | Example |
|---|---|---|---|
| LNA/DNA antimiR-122 | c*c*a*t*t*g*t*c*a*c*a*c*t*c*c | 4 | 11-13 |
| antimiR-122 cRNA (2OM 2PS) | G*GAGUGUGACAAUG*G | 5 | 11 |
| antimiR-122 cRNA (4OM 4PS) | G*G*AGUGUGACAAU*G*G | 5 | 11 |
| antimiR-122 cRNA (6OM 6PS) | G*G*A*G*U*GUGACAA*U*G*G | 5 | 11, 13 |
| antimiR-122 cRNA (8OM 8PS) | G*G*A*G*UGUGACA*A*U*G*G | 5 | 11 |
| antimiR-122 cRNA (10OM 10PS) | G*G*A*G*U*GUGAC*A*A*U*G*G | 5 | 11 |
| antimiR-122 cRNA (12OM 12PS) | G*G*A*G*U*G*UGA*C*A*A*U*G*G | 5 | 11 |
| antimiR-122 cRNA (14OM 14PS) | G*G*A*G*U*G*U*G*A*C*A*A*U*G*G | 5 | 11 |
| antimiR-122 cRNA (OOM 6PS) | G*G*A*GUGUGACAA*U*G*G | 5 | 12 |
| antimiR-122 cRNA (5' mismatch) | A*A*G*GUGUGACAA*U*G*G | 8 | 12 |
| antimiR-122 cRNA (center mismatch) | G*G*A*GUGCAGCAA*U*G*G | 9 | 12 |
| antimiR-122 cRNA (3' mismatch) | G*G*A*GUGUGACAA*G*A*A | 10 | 12 |
| antimiR-122cRNA (5' LNA) | G*G*A*GUGUGACAA*U*G*G | 5 | 12 |
| antimiR-122cRNA (center LNA) | G'G'A'GUGUGACAA*U*G*G | 5 | 12 |
| antimiR-122cRNA (3' LNA) | G*G*A*GUGUGACAA*U*G*G | 5 | 12 |
| antimiR-122 cDNA | g*g*a*gtgtgacaa*t*g*g | 5 | 13 |
| antimiR-122 cDNA (bulge) | g*g*a*gtgtUUUUgacaa*t*g*g | 11 | 13 |

| | | | |
|---|---|---|---|
| Underlined lower-case character: LNA (c represents 5-methylcytosine LNA) Lower-case character: DNA Upper-case character: RNA Underlined upper-case character: 2'-O-methy\| RNA *: phosphorothioate linkage | | | |

An *in vivo* experiment to verify the usefulness of the double-stranded nucleic acid agent according to one embodiment comprising the second strand having a different strand length of unmodified RNA residing in the central part was conducted for more detailed study than that of Example 10.

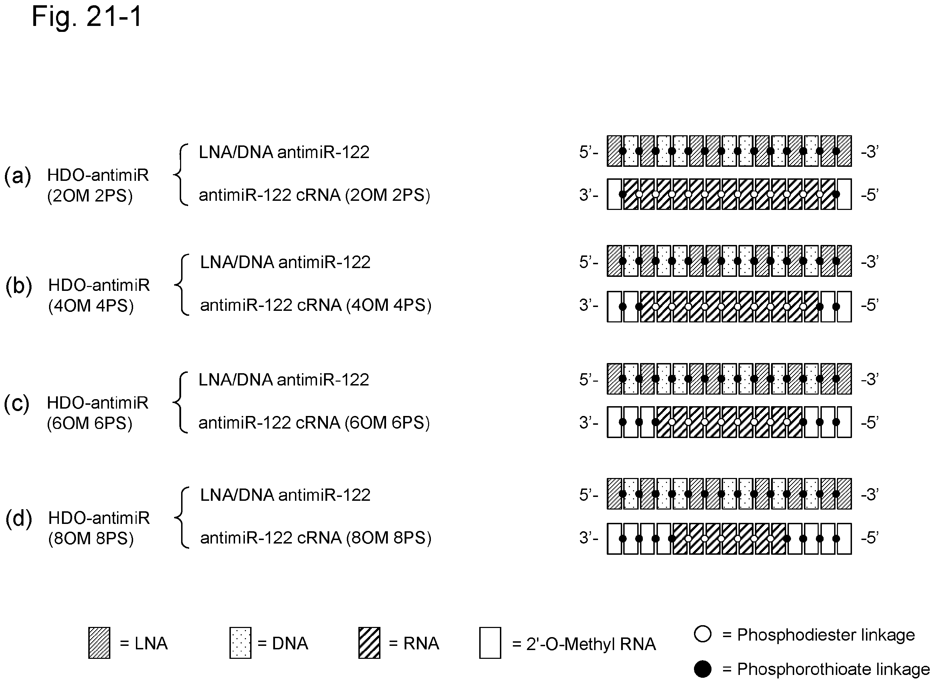

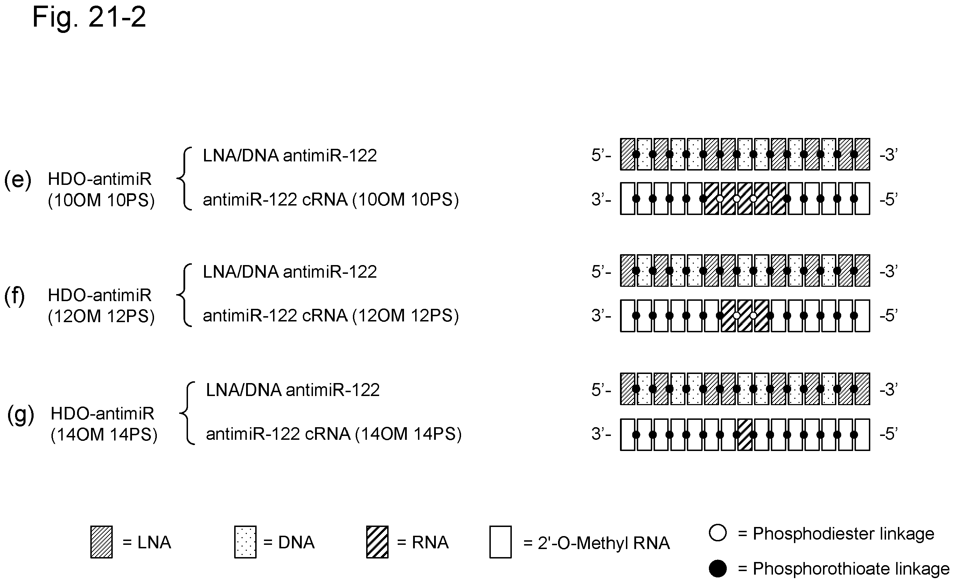

Comparative study was made using double-stranded agents in which the unmodified RNA of the central part was 13 nucleotide length "HDO-antimiR (2OM 2PS), Figure 21a", 11 nucleotide length "HDO-antimiR (4OM 4PS), Figure 21b", 9 nucleotide length "HDO-antimiR (6OM 6PS), Figure 21c", 7 nucleotide length "HDO-antimiR (8OM 8PS), Figure 21d", 5 nucleotide length "HDO-antimiR (10OM 10PS), Figure 21e", 3 nucleotide length "HDO-antimiR (12OM 12PS), Figure 21f", or 1 base long "HDO-antimiR (14OM 14PS), Figure 21g", and the other parts were 2'-O-methyl ribonucleosides comprising phosphorothioate linkages. The sequences, chemical modifications and structures of the oligonucleotides used in Example 11 are shown in Table 2 and Figure 21. The double-stranded agents were prepared in the same way as in Example 1. The target was the same as miR-122 of Example 1. The sequences, chemical modifications and structures of the polynucleotides used in Example 11 are shown in Table 1 and Figure 21. The double-stranded agents were prepared in the same way as in Example 1.

### (In vivo experiment)

Each nucleic acid agent was intravenously injected at a dose of 0.24 µmol/kg to mice (n = 5) through their tail veins. The mice and the miR-122 expression analysis method used were the same as those of Example 1.

### (Results)

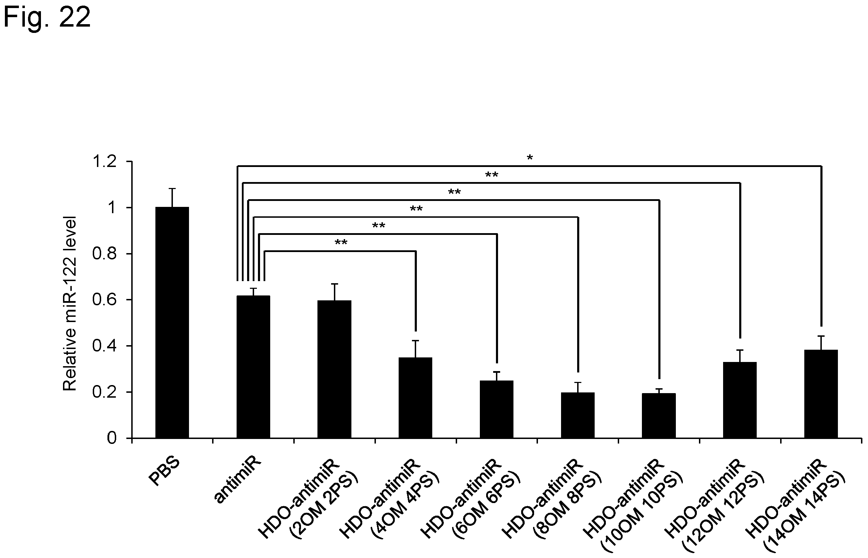

The results of this Example are shown in the graph of Figure 22. The double-stranded agents HDO-antimiR (2OM 2PS), HDO-antimiR (4OM 4PS), HDO-antimiR (6OM 6PS), HDO-antimiR (8OM 8PS), HDO-antimiR (10OM 10PS), HDO-antimiR (12OM 12PS), and HDO-antimiR (14OM 14PS) statistically significantly suppressed the expression of miR-122 as compared with the single-stranded antimiR. Particularly, the "HDO-antimiR (10OM 10PS)" comprising 5 nucleotide length of the unmodified RNA at the central part had the highest miR-122 suppressive effect, demonstrating that there exists a strand length most suitable for the strand length of the unmodified RNA in the central part of the second strand.

### [Example 12]

An *in vivo* experiment was conducted to verify the usefulness of the double-stranded nucleic acid agent according to one embodiment comprising the second strand having a different ability to bind to the first strand.

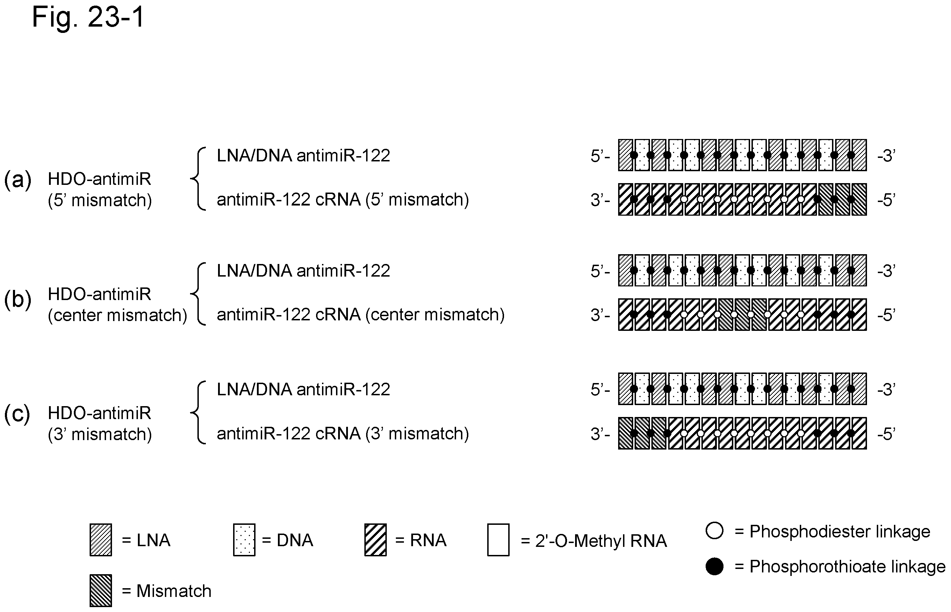

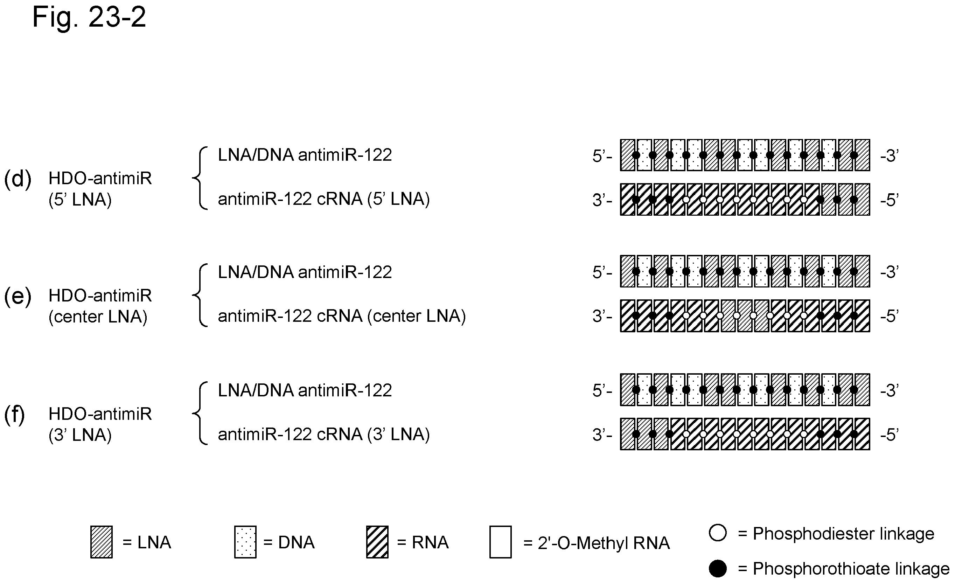

The double-stranded agent "HDO-antimiR (OOM 6PS)" used in Example 8 was used as a reference. Three double-stranded agents comprising the second strand having a sequence (mismatch) noncomplementary to the first strand were synthesized as double-stranded agents comprising the second strand having the reduced ability to bind to the first strand. Specifically, double-stranded agents comprising the second strand having a mismatch of 3 bases at the 5'-terminal, the center, or the 3'-terminal were synthesized as "HDO-antimiR (5' mismatch), Figure 23a", "HDO-antimiR (center mismatch), Figure 23b", and "HDO-antimiR (3' mismatch), Figure 23c", respectively. Also, three double-stranded agents comprising the second strand having an LNA modification were synthesized as double-stranded agents comprising the second strand having the elevated ability to bind to the first strand. Specifically, double-stranded agents having LNA of 3 bases at the 5'-terminal, the center, or the 3'-terminal of the second strand were synthesized as "HDO-antimiR (5' LNA), Figure 23d", "HDO-antimiR (center LNA), Figure 23e", and "HDO-antimiR (3' LNA), Figure 23f", respectively. The target was the same as miR-122 of Example 1. The sequences, chemical modifications and structures of the polynucleotides used in Example 12 are shown in Table 2 and Figure 23. The double-stranded agents were prepared in the same way as in Example 1.

### (In vivo experiment)

Each nucleic acid agent was intravenously injected at a dose of 5.9 nmol/kg to mice (n = 4) through their tail veins. The mice and the miR-122 expression analysis method used were the same as those of Example 1.

### (Results)

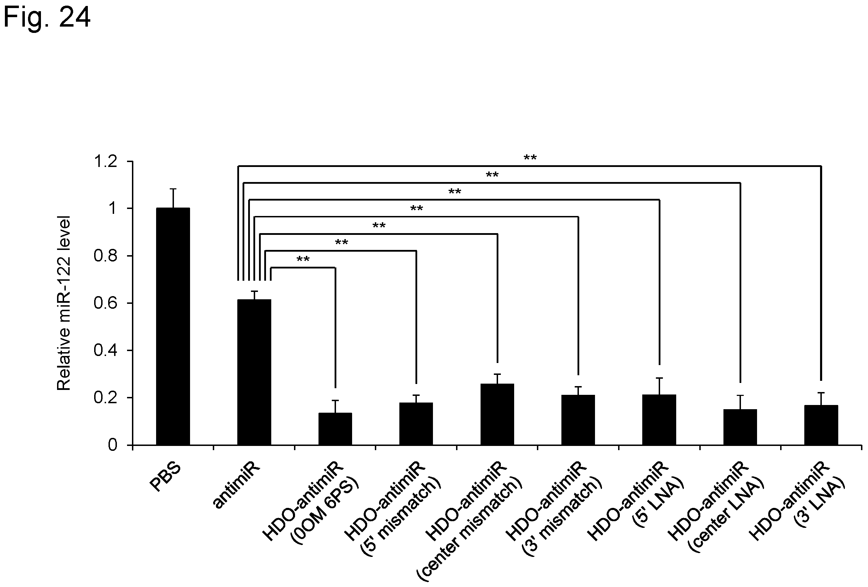

The results of this Example are shown in the graph of Figure 24. All the seven double-stranded agents statistically significantly suppressed the expression of miR-122 as compared with the single-stranded antimiR. Any of the double-stranded agents comprising the second strand having the reduced or elevated ability to bind to the first strand exhibited a high miR-122 suppressive effect, demonstrating that the difference in the ability to bind has less influence on the miR suppressive effect.

### [Example 13]

An *in vivo* experiment was conducted to verify the usefulness of the double-stranded nucleic acid agent according to one embodiment comprising the second strand having DNA different from RNA, and a bulge region constituted by RNA.

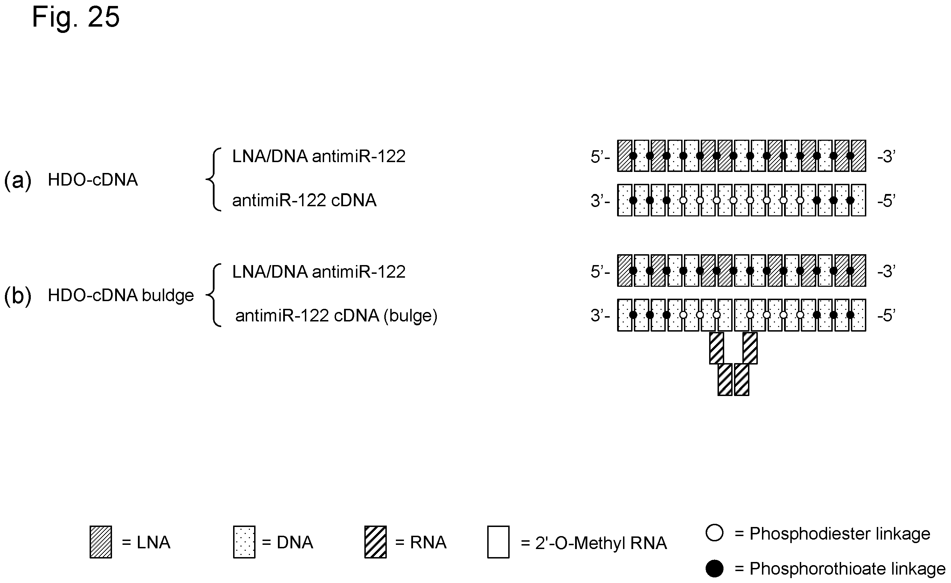

The double-stranded agent "HDO-antimiR (0OM 6PS)" used in Example 8 was used as a reference. Two double-stranded agents comprising the second strand having DNA were synthesized. Specifically, "HDO-cDNA, Figure 25a" comprising the second strand having DNA entirely, and "HDO-cDNA buldge, Figure 25b" comprising the second strand having DNA entirely and a bulge region were synthesized. The target was the same as miR-122 of Example 1. The sequences, chemical modifications and structures of the polynucleotides used in Example 13 are shown in Table 2 and Figure 25. The double-stranded agents were prepared in the same way as in Example 1.

### (In vivo experiment)

Each nucleic acid agent was intravenously injected at a dose of 5.9 nmol/kg to mice (n = 4) through their tail veins. The mice and the miR-122 expression analysis method used were the same as those of Example 1.

### (Results)

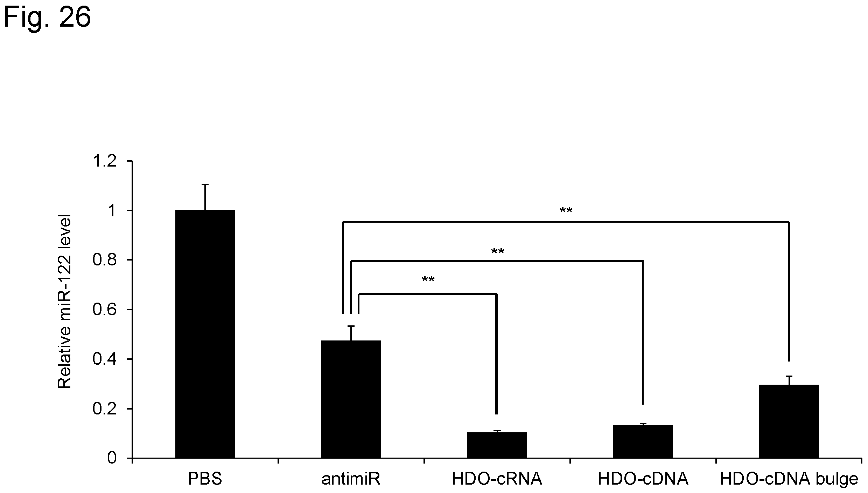

The results of this Example are shown in the graph of Figure 26. All the three double-stranded agents statistically significantly suppressed the expression of miR-122 as compared with the single-stranded antimiR. Any of the double-stranded agents comprising the second strand having DNA replaced for RNA in were found to exhibit a high miR suppressive effect.

### [Example 14]

An *in vivo* experiment was conducted to verify the usefulness of the double-stranded nucleic acid agent according to one embodiment of the present invention in organs other than the liver. Specifically, the usefulness of the double-stranded nucleic acid agent according to one embodiment targeting miR-21, used in Example 6 was verified in the *in vivo* experiment to study its disinhibitory effect on the mRNA expression of the downstream target gene Taf7 of miR-21 in the spleen and the adrenal gland.

"anti-miR" and "HDO-antimiR" used in Example 6 were synthesized. The double-stranded agent was prepared in the same way as in Example 1.

### (In vivo experiment)

Each nucleic acid agent was intravenously injected at a dose of 1.98 µmol/kg or 7.93 µmol/kg to mice (n = 4) through their tail veins. The mice and the Taf7 mRNA expression analysis method used were the same as those of Example 6.

### (Results)

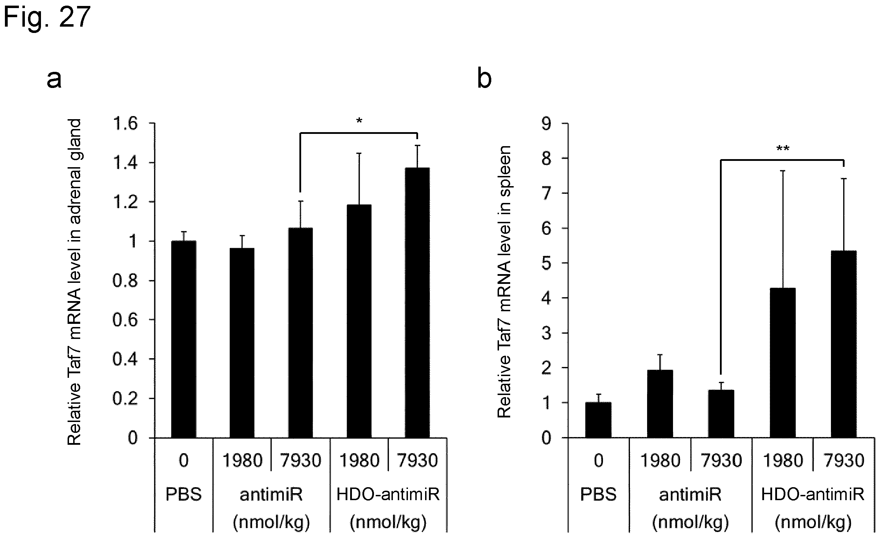

The results of this Example are shown in the graph of Figure 27. The degree of increase in the expression level of the downstream target Taf7 mRNA of miR-21 in both the spleen and the adrenal gland was larger for the double-stranded agent than for the single-stranded agent at both the doses, and was statistically significant for 7.93 µmol/kg of the double-stranded agent. These results indicated that the effect of the double-stranded nucleic acid complex according to one embodiment of the present invention is not specific for the liver and is capable of targeting various organs.

All the publications, patents and patent applications cited herein are incorporated herein by reference in their entirety.

## Claims

1. A double-stranded nucleic acid complex comprising:
a first nucleic acid strand of 6 to 30 nucleotide length that hybridizes to a target miRNA to inhibit a function of the target miRNA; and
a second nucleic acid strand complementary to the first nucleic acid strand, wherein
the first nucleic acid strand is a mixmer comprising a natural nucleoside and a non-natural nucleoside, and
the second nucleic acid strand comprises at least one of one or more modified internucleoside linkages and one or more sugar modified nucleosides.

2. The double-stranded nucleic acid complex according to claim 1, wherein
the second nucleic acid strand
(a) comprises the modified internucleoside linkages consecutively from the 5'-terminal, and comprises the modified internucleoside linkages consecutively from the 3'-terminal;
(b) comprises the modified internucleoside linkages consecutively from the 5'-terminal, and comprises the sugar modified nucleosides consecutively from the 3'-terminal;
(c) comprises the sugar modified nucleosides consecutively from the 5'-terminal, and comprises the modified internucleoside linkages consecutively from the 3'-terminal;
(d) comprises the sugar modified nucleosides consecutively from the 5'-terminal, and comprises the sugar modified nucleosides consecutively from the 3'-terminal; or
(e) comprises the modified internucleoside linkages and the sugar modified nucleosides consecutively from the 5'-terminal, and comprises the modified internucleoside linkages and the sugar modified nucleosides consecutively from the 3'-terminal.

3. The double-stranded nucleic acid complex according to claim 1 or 2, wherein
the second nucleic acid strand
comprises at least four modified internucleoside linkages and/or at least four sugar modified nucleosides consecutively from the 5'-terminal,
comprises at least four modified internucleoside linkages and/or at least four sugar modified nucleosides consecutively from the 3'-terminal, and
comprises one natural ribonucleoside, or 2 to 8 consecutive natural ribonucleosides linked to each other via phosphodiester linkage(s).

4. The double-stranded nucleic acid complex according to any one of claims 1 to 3, wherein at least 50% of internucleoside linkages in the second nucleic acid strand are modified internucleoside linkages.

5. The double-stranded nucleic acid complex according to any one of claims 1 to 4, wherein at least 50% of internucleoside linkages in the first nucleic acid strand are modified internucleoside linkages.

6. The double-stranded nucleic acid complex according to any one of claims 1 to 5, wherein the modified internucleoside linkages are phosphorothioate linkages.

7. The double-stranded nucleic acid complex according to any one of claims 1 to 6, wherein the sugar modified nucleosides comprise 2'-O-methylated sugar.

8. The double-stranded nucleic acid complex according to any one of claims 1 to 7, wherein the mixmer is a BNA/DNA mixmer.

9. The double-stranded nucleic acid complex according to any one of claims 1 to 8, wherein the second nucleic acid strand further comprises a functional moiety having a function selected from a labeling function, a purification function, and a targeted delivery function.

10. A pharmaceutical composition comprising a double-stranded nucleic acid complex according to any one of claims 1 to 9 and a pharmaceutically acceptable carrier.


SEQUENCE LISTING

<110>  National University Corporation Tokyo Medical and Dental University

<120>  Heteroduplex antimiR

<130>  PH-7463-PCT

<150>  JP 2017-129594
<151>  2017-06-30

<160>  11

<170>  PatentIn version 3.5

<210>  1
<211>  22
<212>  RNA
<213>  Mus musculus

<400>  1
uggaguguga caaugguguu ug                                                22


<210>  2
<211>  22
<212>  RNA
<213>  Mus musculus

<400>  2
uagcuuauca gacugauguu ga                                                22


<210>  3
<211>  28
<212>  DNA
<213>  Artificial

<220>
<223>  Synthetic

<400>  3
tggtgcgtat gcgtagcatt ggtattca                                          28


<210>  4
<211>  15
<212>  DNA
<213>  Artificial

<220>
<223>  Synthetic

<400>  4
ccattgtcac actcc                                                        15


<210>  5
<211>  15
<212>  RNA
<213>  Artificial

<220>

<223>  Synthetic

<400>  5
ggagugugac aaugg                                                        15


<210>  6
<211>  15
<212>  DNA
<213>  Artificial

<220>
<223>  Synthetic

<400>  6
tcagtctgat aagct                                                        15


<210>  7
<211>  15
<212>  RNA
<213>  Artificial

<220>
<223>  Synthetic

<400>  7
agcuuaucag acuga                                                        15


<210>  8
<211>  15
<212>  RNA
<213>  Artificial

<220>
<223>  Synthetic

<400>  8
aaggugugac aaugg                                                        15


<210>  9
<211>  15
<212>  RNA
<213>  Artificial

<220>
<223>  Synthetic

<400>  9
ggagugcagc aaugg                                                        15


<210>  10
<211>  15
<212>  RNA
<213>  Artificial

<220>
<223>  Synthetic

<400>  10

ggagugugac aagaa                                                    15

<210>  11
<211>  19
<212>  DNA
<213>  Artificial

<220>
<223>  Synthetic

<220>
<221>  misc_feature
<222>  (8)..(11)
<223>  RNA

<400>  11
ggagtgtuuu ugacaatgg                                                19